# EUROPEAN PATENT SPECIFICATION

(11) **EP 3 162 397 B1**
(45) Date of publication and mention of the grant of the patent: **31.07.2019**
(21) Application number: 15192145.9
(22) Date of filing: 29.10.2015
(51) Int. Cl.: A61M 5/315, A61M 5/20, A61M 5/31

(54) **DOSE SETTING MECHANISM FOR AN INJECTION DEVICE HAVING A DOSE LIMITER**
DOSISEINSTELLMECHANISMUS FÜR EINE SPRITZGIESSMASCHINE MIT EINEM DOSISBEGRENZER
MÉCANISME DE RÉGLAGE DE DOSE POUR UN DISPOSITIF D'INJECTION AYANT UN LIMITEUR DE DOSE

(43) Date of publication of application: 03.05.2017
(73) Proprietor: TecPharma Licensing AG, 3401 Burgdorf (CH)
(72) Inventor: Hostettler, Patrick, 3415 Hasle (CH)

(56) References cited:
- WO-A1-2006/086983

## Description

The invention applies to a dose setting mechanism for an injection device preventing the setting of a dose which exceeds an amount of product present in a cartridge. The dose setting mechanism and the injection device with the dose setting mechanism serves the purpose of dispensing a fluid product, particularly a medicament.

The term "medicament" or "medication" includes any flowable medical formulation suitable for controlled administration through a means such as, for example, a cannula or a hollow needle and comprises a liquid, a solution, a gel or a fine suspension containing one or more medical active ingredients. A medicament can be a composition comprising a single active ingredient or a pre-mixed or co-formulated composition with more than one active ingredient present in a single container. Medication includes drugs such as peptides (e.g., insulin, insulin-containing drugs, GLP-1 containing drugs or derived or analogous preparations), proteins and hormones, active ingredients derived from -or harvested by- biological sources, active ingredients based on hormones or genes, nutritional formulations, enzymes and other substances in both solid (suspended) or liquid form but also polysaccharides, vaccines, DNA, RNA, oligonucleotides, antibodies or parts of antibodies but also appropriate basic, auxiliary and carrier substances.

Injection devices such as injection pens are known in prior art. Individual doses of a product can be set and subsequently dispensed with such injection devices. This procedure can be repeated several times and each time a specific dose can be set and dispensed from the device using the dose setting mechanism. Because the amount of medicament present in the cartridge is limited, namely for example 300 International Units (IU) of insulin, it can happen that a dose is set which is above the amount that is remaining in, and can be dispensed from the cartridge. This could result in that for example 35 IU units are set by the user whereas with the subsequent injection, only 20 IU can be dispensed. The difference of 151U is missing for the therapy and a user of the device might not notice this, which can lead to hazardous adverse dosing.

It is therefore advantageous for the user to have a dosing mechanism including a dose limiting mechanism, which limits the accumulated doses set and dispensed from the device equal to an amount of medication present in the cartridge. The final dose set before emptying the cartridge is limited to the actual amount of medication still present in the cartridge and the user who, for example, intended to set a dose of 35 IU can set a dose limited to the 20 IU that are still available. The user is aware that only 20 IU can be set and dispensed and dispenses the remaining amount of medication required by the therapy, in this case 15 IU, from either a full cartridge inserted in a reusable device or 15 IU from a new and filled disposable device.

Other dosing devices are proposed in prior art, which prevent setting a of a dose which exceeds the amount of product present in the cartridge.

A dose setting limiter for an injection pen with a track and a track follower is described in USRE41956. A dose setting member screws proximally out of the housing versus a non-rotating driver during a dose setting operation. A helical track is located at the outside of the cylindrical driver with a nut member acting as a track follower to count the accumulated doses. The nut member is threadedly engaged with the helical track of the driver and axially guided on the dose-setting member. During dose-setting, the nut member follows the rotations of the dose setting member and moves on the helical track of the non-rotating driver. During dose delivery, the drive member is rotationally coupled to the dose setting member and the position of the nut member on the helical track remains unchanged since there is no relative rotation between the driver and the dose setting member. After repeated dose deliveries, the nut member has moved in the proximal direction towards a fixed stop on the driver and once the nut member abuts the fixed stop at the end of the helical track, further dose setting is prevented and no dose can be set that exceeds the amount of medication present in the cartridge. The length of the helical track thus corresponds to the total doses that can be spent form a cartridge. The nut member, or track follower runs on a helical track that differs from a thread present at the piston rod therefore allowing a different gearing for the piston rod and the track follower. The axis of the helical track is parallel to the longitudinal axis of the injection pen and the track follower moves along the longitudinal axis until the track follower is halted in a stopping position preventing the setting of a dose beyond a limit.

In WO13170392 a dose limiter is described which does not use a helical track and a follower arranged along the longitudinal axis of the pen. A dose setting element is rotated and axially moved out of the proximal end of the pen for setting a dose. A second part or clutch member does not rotate during the setting of a dose, but is coupled to the dose setting member during the dose delivery step and both the dose setting element and clutch member rotate without relative rotations between the two parts. A stop wheel is arranged inside the clutch member on a second axis that is different from the longitudinal axis of the pen. The stop wheel has an outer teething to match an inner teething present on the inside of the dose setting member and can rotate around its own axis, e.g. during dose setting the stop wheel is driven by the dose setting member and due to the eccentric arrangement of the stop wheel and different diameter of the stop wheel, the rotational speed of the stop wheel versus the dose setting member is geared up. During dose delivery the stop wheel does not rotate versus the clutch member or the dose setting member and therefore the cumulative doses set can be counted by the wheel. After a certain number of revolutions of the stop wheel, the stop wheel has reached a stopping position and prevents the further setting of a higher dose whereas the set dose still can be corrected or dispensed. The stop wheel is axially fixed with respect to the housing inside the clutch member and therefore does not need to travel along the longitudinal axis of the pen and is therefore a space saving arrangement compared to the limiter described above in USRE41956. The stop wheel is, however constructed as a wheel inside the clutch member therewith potentially lengthening the clutch member and length of the pen. Additionally the radius of the stop wheel is small, due to the arrangement within the clutch and consequently the compressive forces on the teeth are higher to absorb or transfer a torque to the complementary teeth in the stopping position, therewith putting higher mechanical demands on the materials used for the stop wheel.

In WO 06086983, a dose setting element having a dose limiter is described with a first part that rotates during dose setting and a second part that is rotated with a transmission ratio such that each time the first part has rotated over a certain angle the second part is being rotated over a smaller specific angle until the second part has reached a stopping position and prevents further dose setting. The second part is rotated due to an engagement with the first part during each revolution of the first part, e.g. once per each revolution the first part is axially displaced to enable a short interaction with the second part and thus the second part is slaved together with the first part until the first part axially shifts back to terminate this engagement. The second part counts the cumulative doses set.

In WO 09070911, a dose indicator is presented based on the odometer principle whereby the dose is indicated by two rings that rotate around the central axis of the device and the first ring slaves the second ring intermittently during dose setting. The dose limiting in the described device is separate from the dose indicator and is designed as a stop element that runs on a thread of the drive sleeve rod of the device. During repeated dose setting the stop element moves unidirectional along the axis of the device towards a counter stop.

It is an object of the present invention to present an alternative dose setting mechanism for an injection device compared to the above mentioned mechanisms. It is a further object of the present invention to reduce the size and costs of the device and to increase the reliability of the dose setting mechanism having the dose limiter.

This object is solved with a drive and dosing device according to claim 1. Preferred embodiments are specified in the dependent claims, the description and the figures.

### General description

A dose setting mechanism for an injection device is described which can be integrated into an injection device for delivering at least one dose of medication. The injection device is designed as a single use unit or as a reusable device. The injection device having the dose setting mechanism with the dose limiter delivers the doses from a container, for example a cartridge, containing the medicament. The cartridge comprises a tubular part that is closed with a septum at one end and the circular opening at the opposite end is closed by a stopper to form a compartment for containing the medicament. The septum at the distal end of the cartridge can be pierced by a needle or cannula for delivery of the medication to the patient. The cartridge is connected to the dose setting and delivery mechanism using a cartridge holder. At its proximal end the cartridge holder is connectable to the housing of the injection device. The connection between the housing and the cartridge holder is a releasable connection, for example a bayonet type of connector for a reusable injection device, or it is a permanent or semi-permanent connection, for example a snap-fit connection or an adhesive connection for a disposable or semi-disposable device. The cartridge holder has a tubular shape to encompass the cartridge at the opening from the proximal end. The cartridge holder has a connection site at its distal end for connecting a needle hub with a hollow needle to the cartridge holder. The hollow needle or cannula goes through the needle hub and after piercing the septum of the cartridge connects the inner volume of the cartridge via the needle to the patient.

The tubular housing has a means for viewing the current dose that has been set or corrected, for example a dose scale or an electronic display. The viewing means is, for example, a viewing window with or without a magnifying means. At the distal end of the housing, the cartridge holder is connected to the housing whereas a means for setting or correcting a dose, e.g. a dose-setting element, is located towards the proximal end of the housing. The user can set a dose by rotating and/or axially moving the dose setting element and viewing the dose set from the viewing means. Once a dose has been set, the user can dispense the dose by pressing an actuation button that is located on the housing or on the dose setting element. The actuation element can be located at the outside of the tubular wall of the housing but, preferably, is located at the proximal end of the housing or it is partially enclosed by the dose setting element. The user actuates the device by pressing the actuation button from a non-actuated into an actuated position. The actuation button can actuate the drive and dosing mechanism and finally drive a piston rod in the distal direction and move the stopper in the cartridge accordingly to expel medication from the device. Actuation of the device by pressing the actuation button may alternatively release energy from one or more of the following power packages such as a spring, for example a compression spring, a spiral spring, a helical spring, a coil spring, an extension spring or a torsion spring. The spring can have a single coil or have multiple coils or have a guide pin for guiding the spring forces. Alternatively, the potential energy is released from two separate springs which are coupled to each other in a series or parallel type of arrangement. The coupling can be a direct coupling between the two spring coils or via a third coupling member. The coupling between the two springs can be a permanent coupling or a releasable coupling. The coils of the two springs can have an identical diameter for a series type of arrangement or they are coaxially organized having different diameters. The potential energy needed for driving the injection can also be drawn from other sources such as an expansion of a gas that is compressed in a cartridge or gas that is released from initiating a chemical or electro-chemical reaction. Alternatively, the energy is released from an electronic source driving an electromotor or by volumetric changes of the electro-chemical source itself.

Described above is an arrangement whereby the dose setting element or actuation element is axially fixed with respect to the housing during the dose setting step and the energy for advancing the piston rod originates from a spring, or an electronic source or a motor or an electro-chemical source. In another example, the actuation button and/or the dose setting element move in the proximal direction with respect to the housing during the dose setting and the user returns manually the dose setting element back towards the housing during dose delivery, thereby providing the energy required to advance the piston rod.

The basic principle of the dose setting mechanism having the dose limiter is based on a first part that rotates during the setting of a dose with respect to a second part that does not rotate during the setting of a dose. During dose delivery, both the first part and the second part rotate such that there is no relative rotation between the two parts. The first part rotates in a first direction for increasing a dose and both the first and second part co-rotate in a second direction which is opposite to the first direction during delivery of a dose. If a dose is corrected during the dose setting process, then the first part rotates in the second direction without rotation of the second part. During the dose delivery step, the first and second part are coupled to each other such that no relative rotations are allowed. A third part which limits the setting of a dose beyond a predetermined value is rotated by the first part during the dose setting step. In one example the third part is sandwiched between the first and second part, in another example, the third part surrounds the second part. The third part is driven such by the first part that the third part rotates with a given transmission ratio with respect to the first part. The transmission can be geared up or geared down, e.g. the third part rotates at a higher or lower angular velocity with respect to the first part. The third part can rotate in the same direction as the first part but in an alternative example, the third part rotates in the opposite direction with respect to the first part. During dose delivery, the first, second and third part rotate in the second direction without any relative rotations between the parts and the third part counts the cumulative doses set during the repeated dose setting steps since any axial or rotational displacements of the third part occurring during the dose setting step do not change during dose delivery. The third part preferably rotates from an initial angular position until a final stopping position which corresponds to a predetermined amount of medication present in the reservoir. The second part has a stop member that is operatively coupled to the second part and once the third part has been rotated over a specific or second angle and reached the stopping position, further rotational and/or axial movements of the third part are prevented. In the stopping position for example a protrusion, cam or tooth or ratchet element located on the third part abuts a radial or axial stop or non-complementary surface of the cam, tooth or ratchet element which prevents axial and/or radial shifts of the third and/or second part with respect to each other. In a preferred example, the third part is shifted back and forth during the dose setting step by the first part between a first and second axial position. The first and second axial positions for the third part can be defined by another part, for example the housing or the second part and the axial shifts are limited by flanges, protrusions, axial or radial stops present on the another part. Preferably, the first and second position are a proximal and distal position along the longitudinal axis of the device. During each axial shift of the second part, the second part is rotated over a discrete angular step which corresponds or is correlated to the dosage currently set. For example, each discrete angular step corresponds to 0.5 or 1.0 IU. Thus during dose setting, the third part oscillates between the two axial positions in combination with rotation over discrete angular steps in a zig-zag type of motion. The oscillation of the third part occurs during the dose setting since the third part rotates and axially shifts with respect to the non-rotating and axially fixed second part. During dose delivery, both the second and the third part rotate without any oscillating type of motion between the two. Thus the rotational and axial shifts during the dose setting step are preserved during dose delivery and upon setting the next dose, the third part rotates and axially shifts according to the next dose that has been set. Therefore, the accumulated doses set are represented by the axial and rotational position of the third part with respect to the second part until the axial and rotational steps are halted once the third part has reached the stopping position and the setting of a dose which goes beyond a predetermined amount of medication is prevented. The third part rotates in a single rotation direction during dose setting, preferably corresponding to the rotation direction for increasing a dose. The first part is rotated during dose setting over a first angle, the first angle is defined by both rotation in one direction corresponding to increasing a dose and in the opposite direction for correcting or decreasing a dose. The third part is operatively coupled to the first part such that during each revolution of the first part, the third part is rotated over at least one discrete angular step. The angle of rotation for the discrete angular step being a fraction of the first angle. For example, the third part is rotated over two discrete angular steps per revolution (360 degrees) of the first part. During multiple revolutions of the first part during dose setting steps, the third part is rotated over multiple discrete angular steps until the third part has been rotated over total accumulated angle or second angle.

The third part is operatively coupled to the first part such that rotation of the first part is transferred to the third part. The third part axially shifts between a proximal and a distal position and the shift form one of the two positions to the other position is urged by the presence of coupling members on the third part and the second part respectively. The third part is operatively coupled to the second part such that the interaction of the coupling members urges the third part to axially move or shift. The axial shifts are urged by sloped structures which are present at or part of the coupling members and the interaction of two sloped structures present at two engaging coupling members which slide across each other urge the axial movement of the third part during rotation over the discrete angular step.

The third part preferably shifts back and forth once per revolution of the first part but also other options can be envisaged whereby the third part shifts twice, four times or six times per revolution of the first part.
The rotation of the third part with respect to the second part or the housing or a part connected to the housing during dose setting can be utilized to generate a dose setting click, for example by a two way ratchet system present between the third part and the second part.

The third part axially shifts during dose setting between a proximal and a distal position, preferably the axial shifts are restricted by the second part, for example by flanges, cams or protrusions present on the second part. As an alternative, the axial shifts are restricted by another part, for example the housing. During each axial shift, the third part is decoupled from one of the proximal or distal position and after the axial shift is coupled to the other one of the proximal or distal position. During each axial shift, the third part is rotated over a discrete angular step and the third part is decoupled from one of the proximal or distal position and after the discrete angular step is coupled to the other one of the proximal or distal position. In a preferred embodiment, the coupling and decoupling sequences are designed such that the third part is always in engagement or coupled to the second part, e.g. there is no situation where the third part can freely rotate with respect to the second part during the axial shift. Thus decoupling from one position is not ended before the coupling in the other position has started and there is always an overlap between the two coupling -decoupling sequences.

During the dose setting step, the second part does not rotate with respect to the housing whereas the first part rotates or is rotated by a dose setting member and the first part is operatively coupled to the dose setting member to follow the rotations in the dial up or dial down direction, for example by a drive sleeve. The second part does also not rotate with respect to the third part which is rotated by the first part with a given transmission ratio. During dose delivery, the first second and third part rotate, preferably without any relative rotations between the three parts, and are operatively coupled to a piston rod for advancing the plug in the reservoir. The second part is operatively coupled to the housing such that rotations of the second part in one or first rotation direction, preferably corresponding to the rotation direction for increasing a dose are prevented. In the opposite or second rotation direction for dose delivery, rotation is allowed. Preferably, a reverse lock sleeve is functionally positioned between the second part and the housing whereby the reverse lock sleeve is rotationally coupled to the second part such that rotations of the second part are transmitted to the reverse lock sleeve while axial shift of the reverse lock sleeve are allowed. The reverse lock sleeve is preferably unidirectional rotationally coupled to the housing via a ratchet system, for example a one way ratchet system or a two way ratchet system or a frictional clutch. The unidirectional coupling allows for rotation of the third part in the second rotation direction and prevents rotation in the first rotation direction for increasing a dose. When the third part is in the stopping position and the accumulated doses correspond to the predetermined amount of medication, then increasing a dose is prevented and any rotational torque exerted on the third part by the first part is transmitted to the second part and from the second part to the housing, for example via the reverse lock sleeve thus preventing the further setting of a dose. The rotation of the reverse lock sleeve in the second rotation direction preferably accounts for the delivery click, for example the one-way ratchet system is biased and a teething present on the reverse lock sleeve ratchets over complementary teeth present on the housing.

The first part of the dose setting mechanism is preferably an outer sleeve and the outer sleeve is axially fixed with respect to the housing having a longitudinal axis parallel to the longitudinal axis of the housing, preferably the center of rotation of both axis are identical but alternatives with an eccentric arrangement of both axis can also be envisaged. The outer sleeve is able to rotate or be rotated in the first and second rotation direction during dose setting and rotates in the second rotation direction during dose delivery. The outer sleeve is directly axially coupled to the housing or is operatively coupled to the housing via at least another part, for example via the second part. The outer sleeve has at least two guiding elements located on the inside of the outer sleeve which guide the third part in two ways: on the one hand the two guiding elements transmit a rotational torque from the first part to the third part, on the other hand the two guiding elements axially fixate the third part either in the proximal or in the distal position. The transmission of the torque from the first part to the third results in that the third part is rotated over at least one discrete angular step during each revolution of the first part or outer sleeve. The torque transmitted can, for example, also be used to axially shift the third part in combination with a rotation. Preferably, during each revolution of the first part, there is at least a first phase where the guiding elements transmit torque to the third part and at least a second phase where no rotational torque is transmitted but instead the third part is axially fixed in the proximal or distal position.

The at least two guiding elements are shaped as two arc shaped ribs or protrusions that point towards the central axis of the outer sleeve, and the two arc shaped ribs are, preferably, axially displaced with respect to each other along the longitudinal axis of the outer sleeve. Furthermore, the two arc shaped ribs are, preferably, angularly positioned on the circumference on the inside of the sleeve to leave at least two arc shaped gaps between the two arc shaped ribs. For example four guiding ribs leave four arc shaped gaps between the four arc shaped ribs. Each of the arc shaped ribs has two end surfaces pointing into the circumferential direction and two circumferential surfaces or flanks pointing towards the central axis of the sleeve, one flank on the distal side and one flank on the proximal side.

The third part of the dose setting mechanism is preferably a stop sleeve having a longitudinal axis parallel or coaxial to the longitudinal axis of the housing. The stop sleeve has a steering means preferably designed as a cam or protrusion or ridge located on the outside surface of the sleeve pointing opposite to the central axis of the sleeve. The cam is preferably shaped as a rectangle with first (preferably long) and second (preferably short) surfaces or walls. The first or long surfaces are on the proximal or distal side of the rectangle whereas the two short surfaces are in the circumferential direction. The rib shaped guiding elements of the first part have an end surface that can abut the second surface of the steering means and thereby the rotations of the first element can be transmitted to the third element or stop sleeve. During the dose setting step the stop sleeve oscillates between the proximal and distal position and is kept in one of the two positions due to a sliding engagement between one of the two second surfaces of the steering means and one of the two circumferential surface of the arc shaped ribs.

The steering means on the stop sleeve is preferably arc shaped with an arc shaped length or length of the first surface that is at the most equal to the length of the at least two shaped gaps between the two arc shaped ribs of the first part such that when the third part or stop sleeve is rotated over a discrete angular step by abutment of the end surface of the rib with the second surface of the cam, then the stop sleeve can simultaneously axially shift from one of the proximal or distal position to the other one of the proximal or distal position. Once the stop sleeve is in either the proximal or distal position, the interaction between the first surface of the steering means of the stop sleeve and the circumferential surface of the ribs on the inside of the first part ensure that the stop sleeve is fixed in the distal or proximal position and allows for further rotation of the first part. Once the next end surface of one of the two guiding ribs abuts the second surface of the steering means, either during the same or during the next revolution of the first part (outer sleeve), the stop sleeve is rotated over another discrete angular step and in the meanwhile axially shifted back. This process of shifting back and forth is repeated until the stop sleeve is in its stopping position after being rotated over a specific angle and cannot move axially anymore thereby blocking further dose setting or increasing a dose since the axial shift is needed to ensure that the circumferential surfaces of the guiding ribs of the first part can slide along one of the first surfaces of the cam or steering means. Depending on the number of arc shaped gaps between the arc shaped ribs, the number of shifts back and forth can be programmed, for example with two arc shaped ribs and two arc shaped gaps, the third part will shift once back and forth whereas the third part will shift back and forth twice if there are four arc shaped ribs leaving four arc shaped gaps.

In a preferred embodiment, the third part or stop sleeve is coaxially arranged around the second part which is designed as a coupling sleeve and the third part is axially slidable between a proximal and a distal position. The second part or coupling sleeve is equipped, for example, with at least two flanges or protrusions or rims or the coupling sleeve has a circumferential recess and the edges of the flanges, rims or recess limit the axial shift of the third part between the two positions. For example a proximal flange and a distal flange are operatively coupled to the second part such that in the gap between the two flanges the third part or stop sleeve can shift between the two flanges. The flanges, protrusions or recesses on the second part can be shaped as integral part or are separate parts that are assembled to the main body of the second part. Functionally, the flanges, protrusions or recesses act as if they are an integral part of the second part.

In another embodiments, the third part surrounds the second part for example the third part is a hollow cylinder surrounding the second part and the cylinder is at least partially closed at the proximal and distal end. The longitudinal axis of the second part penetrates through the proximal and distal end surfaces of the third part. The first and third coupling members that will be described later are located on the inside of the cylinder and restrict the axial movements of the third part with respect to the second part which is axially fixed.

In yet another embodiment the third part is a sleeve that is coaxially arranged on the second part and has a track follower, for example a guide pin on the inside of the sleeve which follows a track present on the outside of the second part. For example, the track can be a zig-zag groove circumferentially arranged on the outside of the second part. The axial movements of the third part between the proximal and distal position are restricted by the interaction of the track follower in the track.

The third part or stop sleeve has at least one first coupling member which can be coupled or decoupled to a second coupling member preferably present at the proximal flange, recess or protrusion. Preferably, the at least one first coupling member is located at the proximal end or rim of the stop sleeve. Furthermore, the third part or stop sleeve has at least one third coupling member present at the distal end which can be coupled or decoupled to a fourth coupling member present at the distal flange, rim or protrusion on the second part. The relative rotations between the stop sleeve and the second part are restricted when the at least one first coupling member and the second coupling member are engaged or coupled or when the at least one third coupling member and the fourth coupling member are engaged or coupled. Thus the relative rotations between the third part and the second part are limited when the third part is in the proximal or distal position and the rotation of the third part is allowed during the axial shift from the proximal position to the distal position or vice versa from the distal position to the proximal position.

The at least one first coupling member located at the third part preferably comprises at least one sloped structure, for example a tooth having two sloped structures. The at least one first coupling member is preferably located at the proximal rim of the stop sleeve and pointing in the proximal direction. The at least one first coupling member is preferably shaped as a symmetric or an asymmetric tooth and the tooth matches a complementary structure (second coupling member or sloped structure) present at the proximal flange of the second part and which points in the distal direction. Once the tooth and the complementary structure are engaged, e.g. due to an axial shift, a form fit or abutment of flanks of the teeth restrict the relative rotations. Once the third part is engaged with the second part and the third part is rotated with respect to the second part, then the two coupling structures are brought out of engagement whereby the slopes or flanks of the teething slide over each other and the third part shifts from the proximal position towards the distal position. The third part has the at least one third coupling structure, preferably present at the distal end of the stop sleeve and pointing in the distal direction, preferably a loped structure shaped as at least one tooth. The tooth is designed as a symmetric or an asymmetric tooth which matches a complementary toothing present at the distal rim, flange or recess of the second part. Once the third part is in the distal position, the teeth of the third and fourth coupling structure are in engagement or in the coupled position and limit the relative rotation between the third part and the second part. If the third part is rotated with respect to the second part, then the relative rotation and engagement of the sloped structures or flanks of the teething push the third part or stop sleeve from the distal position towards the proximal position. The coaxial arrangement of the third part around the second part has the advantage that the first and third coupling structures are located at a relatively long distance from the rotational axis of the pen, thus torques that need to be transmitted from the third part to the second part via the coupling structures result in relatively low contact forces or contact pressures, for example between the flanks of complementary teethings.

The at least one first coupling member and the second coupling member are decoupled from the coupled position when the at least two guiding elements of the first part rotate the third part over the discrete angular step. During the decoupling, the third part or stop sleeve axially shifts for example from the proximal to the distal position and the at least one third coupling member is coupled to the fourth coupling member, preferably located at the distal flange of the second part. During the axial shift the arc shaped cam of the third part simultaneously shifts through one of the at least two arc shaped gaps between the two guiding element of the first part. Thus the sequence of rotational engagement between the first part and the third part via the end surface of the guiding ribs, the decoupling of the at least one first coupling structure from the second coupling structure that initiates and urges the axial shift from the proximal position to the distal position and the coupling of the at least one third coupling member to the fourth coupling member is such that the arc shaped cam or steering means of the third part simultaneously passes one of the at least two arc shaped gaps between the two guiding elements of the first part. Once the third part or stop sleeve is in either the proximal or distal position, the interaction between the first surface of the steering means of the stop sleeve and the circumferential surface of the ribs on the inside of the first part ensure that the stop sleeve is fixed in the distal or proximal position. Preferably, within the same revolution of the first part, the sequence is repeated in reverse order and the third part shifts back from the distal to the proximal position and is rotated over another discrete angular step. In a preferred embodiment, the third part shifts one time back and forth during each revolution of the first part but other options can easily be envisaged whereby the third part shifts twice or three times per revolution of the first part. As a consequence, the transmission ratio between the rotations of the first part and the third part can be selected and adapted to the specific requirements needed. The specific arrangement and number of the at least two guiding elements of the first part need to be accommodated accordingly.

The third part or stop sleeve rotates during each axial shift over a discrete angular step until the accumulated angular steps are equal to the specific angle. Once the stop sleeve has been rotated over the specific angle, the axial shift is prevented and thereby further dose increase is prevented and rotation of, for example, a dose setting element is prevented. The torque exerted on the first element in the dial up direction is redirected to the housing via a cascade of elements, for example via the third and second part to the reverse lock sleeve and finally to the housing as described above. The axial shifts back and forth are halted in the stopping position due to the fact that the at least one first coupling member cannot be coupled to the second coupling member in the proximal position of the stop sleeve and/or the at least one third coupling member cannot be coupled to the fourth coupling member. In a preferred embodiment, the second coupling member is designed as a toothing present at the proximal flange and pointing in the distal direction. One or more of the teeth of the toothing can be filled or have a different shape that is not complementary to the tooth of the at least one first coupling member, thus forming the stop element. Due to the structural mismatch there is an obstacle (no form fit) between the first and second coupling members in the stopping position and consequently the axial shift of the stop sleeve is prevented. The stop member can also be located at the distal flange of the second part where the fourth coupling member is preferably designed as a toothing structure and also one or more of the teeth of the fourth coupling member can be filled, closed or shaped in a non-complementary manner to the at least one third coupling member such that a distal shift of the stop sleeve from the proximal position is prevented, thereby blocking further rotation of the stop sleeve over a discrete angular step. Thus once the at least one first or third coupling member reach the stop element which is present at the distal and/or proximal flange of the second element, further axial shifts and consequently rotation over discrete steps are prevented thereby preventing the further setting of a dose. A rotation in the opposite direction, corresponding to reducing a set dose is still allowed. The torque is transferred from the first part to the third part via at least one steering means and from the third part to the second part via one of the first or second coupling members and from the second part to the housing, either directly or via another part such as a reverse lock sleeve.

In a preferred embodiment, the coupling and decoupling of the at least firt and the at least third coupling structures is such that the second part is always coupled to the second part, or in other words, there is no situation where the third part can freely rotate with respect to the second part. This ensures a reliable limiting mechanism since the third part is always in engagement with the second part and even external forces such as dropping the device, temperature shocks, penetration of fluids or mis-use which might lead to uncontrolled rotation of the third part with respect to the second part leading to erroneous dose limiting is prevented.

The dose setting and limiting mechanism device described above is, preferably, integrated in an injection device for delivering the medication. The Injection device has a dose setting element that the user grabs and rotates and/or axially displaces with respect to the housing to set the desired dose. A dose scale or scale drum is operatively coupled to the dose setting element such that the user can read the current dose set. The user rotates the dose setting element in one rotation direction for increasing a dose and in the second or opposite direction for correcting the dosage that user can read, on the scale drum, preferably through a window in the housing. The first part or outer sleeve is operatively coupled to the dose setting element such that rotations of the dose setting element are transferred to the first part. The operative coupling can be a direct coupling between the dose setting element and the first part or via another part, for example a drive sleeve. The first part is rotationally coupled to the drive sleeve and, preferably axially slidable coupled to another. The injection device preferably has an actuation button which can be shifted by the user from a non-actuated into an actuated position. During actuation, the drive sleeve is coupled to the second part and therewith the first part, the second part and the third part are brought into a rotationally secured engagement and start rotating, preferably in the second rotation direction once the energy of the actuation button and/or power package is released. The second part or coupling sleeve is operatively engaged with a piston rod such that the rotation of the coupling sleeve results in axial advancement of the piston rod. The distal end of the piston rod preferably has a flange that abuts the plug in the reservoir and advancement of the piston rod advances the plug to expel medication from the reservoir.

In one embodiment, the second part or coupling sleeve is splined to the piston rod in a non-rotatable but slidable engagement, for example through longitudinal grooves in the piston rod that match keys on the inside of the coupling sleeve. The piston rod has furthermore a threading on the outside that matches an internal threading of the housing and rotation of the coupling sleeve results in rotation and advancement of the piston rod through the housing.

In yet another embodiment, the piston rod has a threading, preferably an external thread that is in a threaded engagement with an internal thread of the second part or coupling sleeve. The piston rod is keyed to the housing in a non-rotatable but sliding engagement and rotation of the coupling sleeve slides and advances the piston rod through the housing.

The dose limiter can be programmed by positioning the third part at a specific position with respect to the teethings of the second part. Thus a method of programming the dose limiting is part of the present invention. For example placing the third part in the stopping position and rotating the first part in the direction for decreasing a dose rotates the third part back to a desired starting position. Alternatively, the third part is mounted during the assembly of the device at the desired position, with the programming method, the dose setting can be restricted to a predetermined of medication, the predetermined amount of medication can be equal or below the volume of medication present in the reservoir. Thus setting can, for example, be restricted to half the amount of medication present in the reservoir.

A dose limiting mechanism for an injection device for injecting at least one dose of medication from a reservoir comprising a housing with a distal and a proximal end, the housing having a longitudinal axis whereby the reservoir is located at the distal end of the housing. The mechanism further comprising a first part which is rotated over a first angle during setting of a dose, a second part which does not rotate during setting of a dose and a third part which is rotated during the setting of a dose by the first part with a given transmission ratio. The third part is operatively coupled to the first part such that the third part is rotated by the first part over at least one discrete angular step which is a fraction of the first angle.
The rotation axis of the first, second and third part are approximately around and/or parallel to the longitudinal axis of the housing. The second part comprises a stop member which prevents further rotation of the third part when the third part has been rotated over a second angle, thereby preventing the setting of a dose which exceeds a predetermined amount of medication from the reservoir. The third part is urged to axially shift back and forth during dose setting between a proximal and a distal position due to engagement of coupling members present at the third part and the second part whereby during each axial shift the third part is rotated over one discrete angular step. Furthermore, during each angular step the third part, in its proximal or distal position is coupled to, or decoupled from the second part and is simultaneously coupled to, or decoupled from, the second part to the other one of the proximal or distal position. The second part is operatively coupled to the housing such that rotations of the second part in one rotation direction corresponding to dose increase are prevented and rotations in the opposite direction are allowed, preferably by a reverse lock sleeve that is functionally positioned between the second part and the housing, and whereby the reverse lock sleeve is rotationally coupled to the second part and coupled to the housing via a one-way ratchet system.
The first part of the dose setting mechanism is an outer sleeve axially fixed with respect to the housing having at least two guiding elements and the at least two guiding elements guide the third part during each revolution such that the third part is rotated over the discrete angular step and is axially secured in the proximal or distal position. The at least two guiding elements are preferably arc shaped guiding ribs circumferentially arranged on the inside of the outer sleeve and the at least two arc shaped ribs are positioned axially displaced along the longitudinal axis with respect to each other and are angularly positioned with respect to each other to leave at least two arc shaped gaps between the at least two arc shaped ribs. The third part of the dose setting mechanism preferably is a stop sleeve, which has a steering means which can be operatively coupled to one of the at least two arc shaped ribs of the outer sleeve such that the stop sleeve is rotated during each revolution of the first part and axially secured either in the proximal or distal position. The steering means preferably is an arc shaped cam fixed on the outside of the stop sleeve with an arc length at the most equal to the arc shaped gap of the outer sleeve such that the stop sleeve can be rotationally coupled to the first part by one of the at least two arc shaped ribs and whereby the arc shaped gaps allow for axial shifts of the arc shaped cam. The third part is preferably coaxially arranged around or surrounds the second part and/or is located between a proximal and a distal flange of the second part such that the axial movements of the third part between the proximal position and the distal position are restricted by the flanges, or the third part is coaxially arranged around the second part and whereby the second part has a track and the third part a track follower such that the axial movements of the third part between the proximal position and the distal position are restricted by the track and the track follower. The third part of the mechanism preferably has at least one first coupling member which can be coupled or decoupled to a second coupling member located at the second part whereby the third part has at least one third coupling member which can be coupled or decoupled to a fourth coupling member located at the second part such that relative rotations between the third part and the second part are restricted when the third part is in the proximal or distal position and the first and second coupling structures are coupled or the third and fourth coupling structures are coupled. The at least one first coupling member of the third part comprises at least one tooth located at the proximal rim and pointing in the proximal direction which fits into the second coupling member which is designed as a teething located at the proximal flange of the second part and which points in the distal direction, and whereby the third coupling member of the third part comprises at least one tooth located at the distal rim pointing in the distal direction which fits into the fourth coupling member designed as a toothing located at the distal flange of the second part and which points into the proximal direction. The first and second coupling members are decoupled from the coupled position when the at least two guiding elements rotate the third part over a discrete angular step, whereby the third and fourth coupling members are coupled from the decoupled position, whereby the third part axially shifts from the proximal to the distal position. The proximal or distal flange comprises preferably the stop member which prevents axial shifts of the third part when the first or third coupling structure abuts the stop member.

### Legends to Figures

- Figure 1:: Dose limiting mechanism according to a first embodiment of the invention comprising a first part, a second part and a third part.
- Figure 2:: Functioning of the dose limiting mechanism during dose setting, first interaction between the three parts during a revolution of the first part. The sleeve of the first pat has been removed for clarity, only the two guiding elements of the first part are shown.
- Figure 3:: Functioning of the dose limiting mechanism during dose setting, second interaction between the three parts during the revolution of Figure 2.
- Figure 4:: Functioning of the dose limiting mechanism during the setting of the last dose.
- Figure 5:: Exploded view of an injection device having the dose limiting mechanism as shown in Figures 1 to 4.
- Figure 6a:: Cross section of the injection device of Figure 5, no dosage set.
- Figure 6b:: Cross section of the injection device of Figure 5, no dosage set.
- Figure 7:: Cross section of the injection device of Figure 5, dose set and dose setting element rotated over 360°.
- Figure 8:: Cross section of the injection device of Figure 5, release button in actuated position.
- Figure 9:: Cross section of the injection device of Figure 5, after injection and advancement of the piston rod.
- Figure 10:: Cross section of the injection device of Figure 5, in dose limiting position, stop last dose activated.
- Figure 11a:: Dose limiting device according to a second embodiment of the invention, dose setting. The sleeve part of the first and second part have been removed for illustrative purposes, see also Figure 11c.
- Figure 11b:: Dose limiting device according to a second embodiment of the invention, dose limiting position.
- Figure 11c:: Dose limiting device according to a second embodiment of the invention, cross section.
- Figure 12:: Dose limiting device according to a third embodiment of the invention.
- Figure 13:: Dose limiting device according to a fourth embodiment of the invention.

### Detailed description

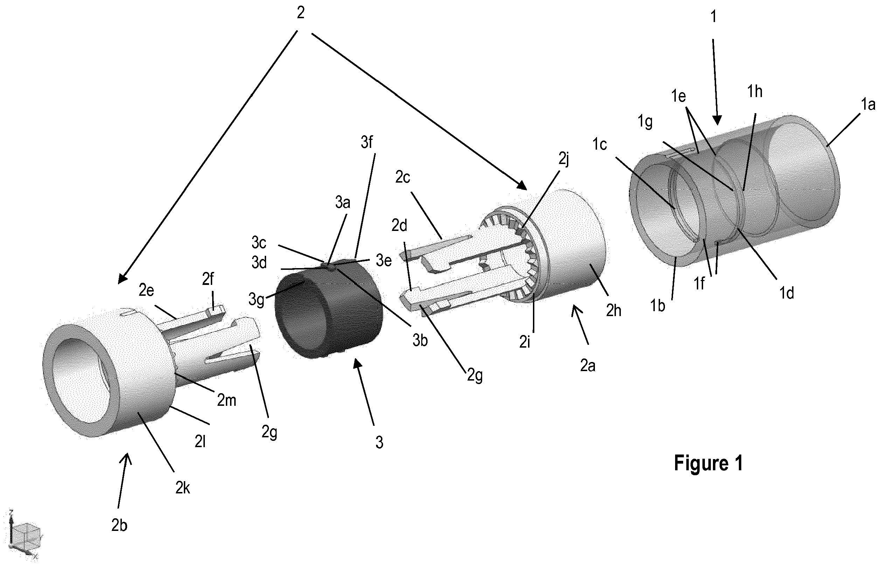

Parts of the dose limiter: The basic parts of the dose limiter are described below and shown in Figure 1: The first part (1) or outer sleeve is axially fixed but rotatable with respect to a housing (4) that will be described below. The first part is a sleeve with a proximal rim (1a) and a distal rim (1b) and having a longitudinal axis parallel to the main axis of the housing. On the inside surface of the first part there are at least two guiding elements (1c, 1d) preferably arc shaped guiding ribs and the at least two guiding ribs are axially displaced relative to another. Each guiding rib has two end surfaces (1e, 1f) and each has two circumferential surfaces (1g, 1h, only indicated for one guiding rib in Figure 1). One of the two circumferential surfaces (1g) is on the distal side, the other of the two circumferential surface (1h) is on the proximal side. The first part (1) rotates during dose setting in the first rotating direction for increasing a dose and in the second rotation direction which is opposite to the first rotation direction for decreasing a set dose. During dose delivery, the first part rotates in the second rotation direction. After dose setting with or without dose correction the first part (1) is rotated over a first angle.

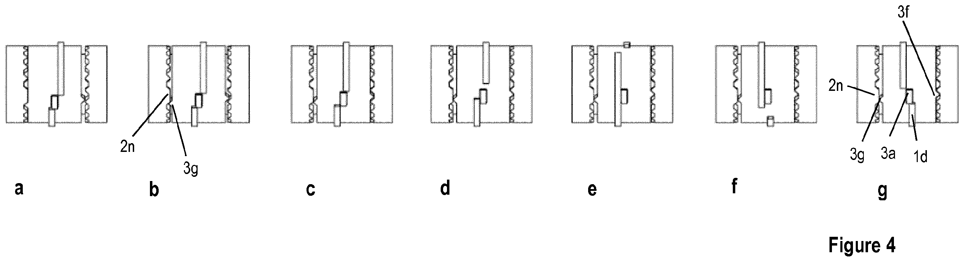

The second part (2) is shown exemplary as being composed of a proximal part (2a) and a distal part (2b). The proximal part (2a) and distal part (2b) comprise a proximal sleeve (2h) and distal sleeve (2k). Functionally, the distal and proximal part behave as a single part and are for, for example, for the manufacturing process, made as two separate parts that can be joined together The proximal part has connecting arms (2c) that point in the distal direction which can be assembled with connecting arms (2e) of the distal part (2b). During the connection process, the connector arms (2c, 2e) can flex due to the presence of a wedge shaped gap (2g) and can snap fit together using the connectors (2d, 2f) that are attached to the ends of the connector arms. Once joined together, the second part is a dog-bone shaped cylinder with a larger diameter of the proximal and distal sleeves (2h, 2k) and a connecting region with a smaller diameter. The proximal sleeve (2h) of the proximal part (2a) ends at a distal step or end surface (2i) onto which a circumferential toothing (2j) projects in the distal direction. The distal sleeve (2k) has a proximal step or surface (21) having a circumferential toothing (2m) projecting in the proximal direction. One of the two toothings (2j, 2m), for example the distal toothing (2m) has a stop member (2n, Figure 4) which is designed as one or more closed teeth of the toothing (2m).

The third part or stop sleeve (3) is shaped as a sleeve with a longitudinal axis parallel to the longitudinal axis of the housing. The stop sleeve (3) is concentrically arranged around the second part (2), preferably around the smaller diameter region of the dog-bone shaped second part (2). In this example, the third part (3) is formed as a single part and the second part (2) is made of two separate parts that are snap-fitted together but other options can easily be envisaged, for example the third part (3) can be made of two arcs that can be joined together around the second part. The stop sleeve can axially slide on the second part and the axial shift is restricted by the proximal surface (2i) and the distal surface (21) of the second part (2), e.g. the third part can shift back and forth on the smaller diameter region of the dog-bone shaped second part (2).

The third part or stop sleeve has a steering means or cam (3a) on the outside surface pointing opposite to the central axis of the sleeve. Preferably the steering means is shaped as a rectangle but other forms such as circles, triangles or squares and the like can easily be envisaged. The rectangular shaped cam has two second surfaces or short surfaces (3b, 3c) and two first or long surfaces (3d, 3e), the latter are either on the proximal side (3e) or on the distal side (3d). The third part or stop sleeve has a first coupling member (3f), present at the proximal rim of the stop sleeve and shaped as at least one tooth that points in the proximal direction. The first coupling member (3f) is complementary to the second coupling member or circular toothing (2j), and the tooth (3f) and teething (2j) can be designed as having symmetric or asymmetric teeth. Furthermore, the third part or stop sleeve has a third coupling member (3g), preferably shaped as at least one tooth pointing towards the distal direction and which is located at the distal rim of the stop sleeve. The third coupling member (3g) is preferably complementary to the fourth coupling member or circular toothing (2m).

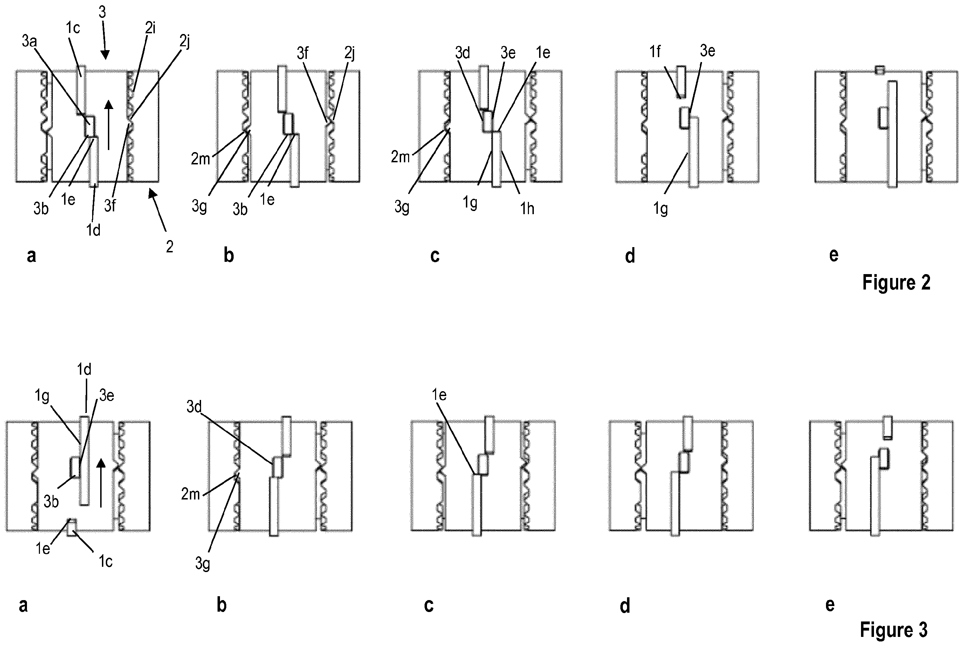

The functioning of the above described embodiment parts is described below and shown in Figure 2, for illustrative purposes, the sleeve part of the first part (1) or outer sleeve has been removed. The first part (1) rotates in the dial up direction as indicated by the arrow. The end surface (1e) of the guiding rib (1d) abuts the second surface (3b) of the steering means (3a). The third part is in the proximal position and the first coupling member (3f) is coupled to the second coupling member (2j) of the second part (Figure 2a). Torque in the dial up direction is transmitted from the first part (1) to the third part (3) via the arc shaped guiding rib (1d). The flanks of the tooth of the first coupling member (3f) slides across the teething (2j) of the second coupling member of the second part. The second part is rotationally secured with respect to the housing and consequently the mutually sliding surfaces of the teeth (3f) and (2j) axially shift the third part (3) from the proximal position towards the distal position (Figure 2c). During the axial shift, the coupling comprising the first and second coupling members (3f, 2j) is decoupled and on the opposite side the third coupling member (3g) is coupled to the fourth coupling member (2m). During the axial shift of the third part (3) towards the distal position, the end surface (1e) of the guiding element of the first part (1) slides across the short surface (3b) of the steering means (3a). Once the axial slide of the third part (3) has been completed (Figure 3c), the rotation of the first part or outer sleeve can continue and the long proximal surface (3e) of the steering means (3a) can slide along the distal circumferential surface (1g) of the arc shaped guiding rib (1d), Figure 1d. Once the shift has been completed, the interaction of the surfaces (1g,3e) axially secures the third part (3) in the distal position. After a rotation of a certain angle depending on the number of guiding elements present on the inside of the outer sleeve, in this example after 180°, the next arc shaped guiding rib (1c), which is in this case positioned axially distally from the arc shaped guiding rib (1d), approaches the steering means (3a, Figure 3a). The end surface (1e) of the guiding rib (1c) abuts the short surface (3b) of the steering means (3a, Figure 3b). The stop sleeve (3) is coupled in the distal position by the coupling (2m,3g) and the rotation of the outer sleeve (1) is transmitted to the stop sleeve (3). The coupling (2m, 3g) is moved towards the decoupled position due to the interaction of the flanks of the toothing, and the stop sleeve (3) is urged to move towards the proximal position (Figure 3c). Once the steering means has passed the end surface (1e) of the guiding rid (1c), the long surface of the steering means on the distal side (3d) can slide along circumferential surface (1g) of the guiding rib (Figure 3e). The stop sleeve is now secured in the proximal position and the coupling between the first and second coupling members (2j, 3f) is coupled. During each axial shift back and forth, the stop sleeve (3) is rotated over one discrete angular step. The axial shifts occur during dose setting, e.g. during dial up and dial down of a dosage. The dial down or dose correction sequence is analogous to the steps described above, e.g. the outer sleeve (1) is rotated in the opposite direction and the end surface (1f) of the guiding rib abuts the short surface (3c) of the steering means to start the simultaneous decoupling - axial shift and coupling sequences (Figure 2d).

After a predetermined number of discrete angular steps, the accumulated angle rotated by the stop sleeve equals the rotation over a specific angle or second angle and the further setting of a dose is prevented (Figure 4). In Figure 4a, the configuration is shown whereby the stop sleeve (3) is in the distal position and the tooth of the third coupling member (3g) is in the last angular position before the stop member (2n). Rotation of the first part shifts the stop sleeve back to the proximal position due to the interaction between the steering means (3a) and the end surface of the guiding rib (Figures 4b,4c and 4d). The stop sleeve is secured in the proximal position and the first and second coupling members (3f, 2j) are in the coupled position (Figure 4e). Once the end surface of the guiding rib(1d) abuts the steering means (3a), the first and second coupling members (3f,2j) intend to move into the decoupled position and the torque is transferred between the flanks of the tooth and teeth of the first and second coupling members. The stop sleeve (3) wants to shift in the distal direction and rotate over the discrete angular step and couple the third coupling member (3g) to the fourth coupling member (2m). The axial shift is prevented since the tooth of the third coupling member cannot fit in the stop member (2n) and, as a consequence, the rotation of the first part or outer sleeve (1) is blocked by the stop sleeve. The end surface (3d) of the steering means (3a) axially cannot pass the arc shaped guiding rib (1d, Figure 4g). A correction of the dose is allowed and can be visualized by the reverse order of the sequences presented in Figure 4. In the stopping position whereby the third coupling member (3g) abuts the stop member (2n), the rotational torque is transmitted as follows in the example as shown in Figure 4: From a dose setting element that is operatively coupled to the first part (1), to the stop sleeve (3) via the steering means (3a) and via the first coupling member (3f) to the second part via teething (2j). The torque is finally transmitted to the housing via an operative coupling to the housing, for example through a one-way ratchet system or a reverse lock sleeve that will be discussed later.

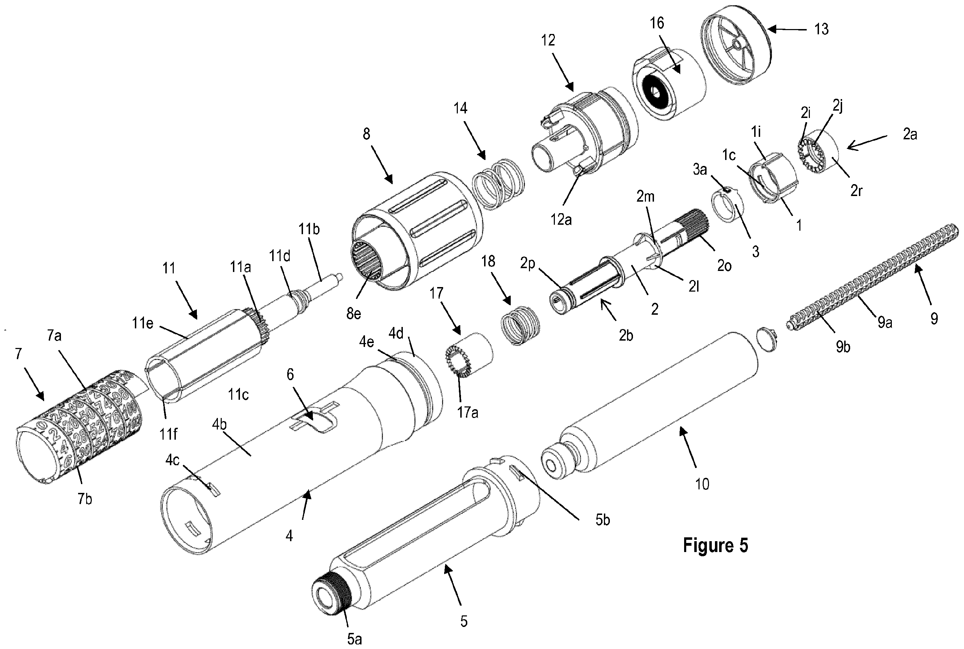

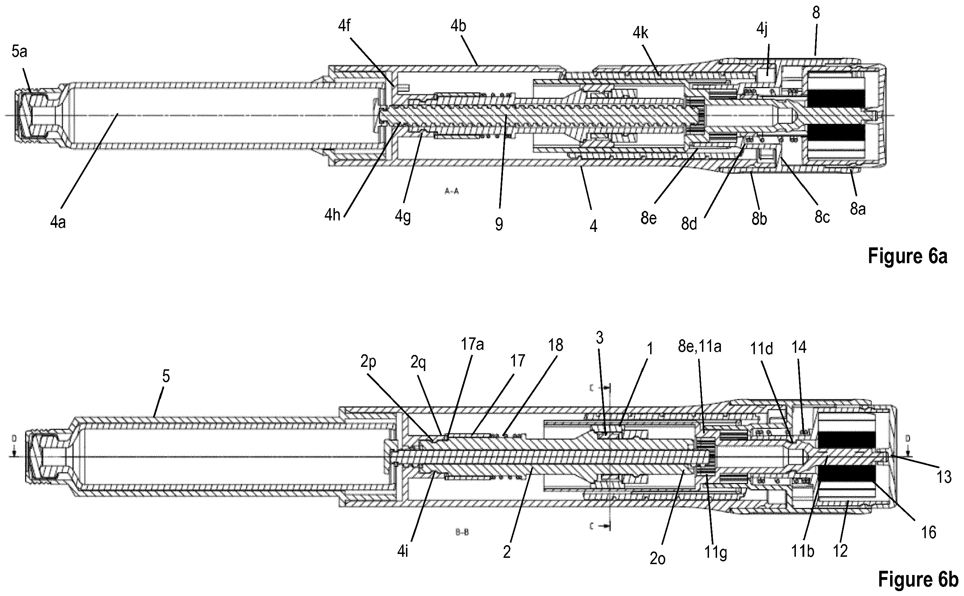

An exploded view of an injection pen having the dose setting mechanism according to a first embodiment of the invention is shown in Figure 5 and a cross section of the assembled pen before a dose has been set in Figure 6. The injection pen comprises a tubular housing (4) having a longitudinal axis (4a) with an outer housing or housing sleeve (4b). The tubular housing has a distal end designed for attaching a cartridge holder (5), for example suing a snap-fit connection (4c). The outer housing sleeve (4b) has a viewing window (6) for viewing the numerical values (7a) printed on a scale drum (7). On the proximal end, the housing ends in a circumferential rim (4d) for engaging a dose setting element (8) via notch (4e) at the proximal end of the housing. In the distal section of the housing, the outer housing sleeve (4b) has a circumferential rim (4f) pointing towards the center of the housing for connecting an inner housing sleeve (4g) to the outer housing sleeve (4b). An internal thread element (4h, Figure 6) is present on the inside of the inner housing sleeve (4g) for engaging a piston rod (9) having an outer threading (9a) and longitudinal grooves or keyways (9b). The distal end of the outer housing sleeve (4b) is designed to receive the cartridge holder (5) containing the cartridge (10). Furthermore, the outer housing sleeve (4b) has internal thread segment (4k) for engaging an outer threading (7b) of the scale drum (7).

The cartridge holder (5) has a tubular shape and at the proximal part, protrusions (5b) are present that match the snap fit connector (4c) of the housing (4). At the distal end the cartridge holder has a needle connector (5a) for attaching a needle having a needle hub to the device (not shown in the drawings). The needle hub can be connected to the needle connector (5a) using a click-on or screw type movement. The needle or cannula goes through the needle hub and while attaching the needle hub pierces a septum present at the distal end of the cartridge (10) and thereby connecting the inside of the cartridge via the needle to the patient. The cartridge (10) has a cylindrical shape which fits into the proximal end of the cartridge holder (5). At the proximal end, a plug, piston or stopper (not shown in the Figures), preferably made from a rubber material closes the cartridge and the medication is present between the plug and the septum at the distal end of the cartridge.

A dose setting element (8) is located at the proximal end of the housing (4) having an outer sleeve with a distal rim section (8b) and a proximal rim section (8a). The proximal and distal rim sections are joined at a position where a rim (8c) points towards the center of the housing and the rim (8c) connects the outer housing sleeves (8a, 8b) with an inner housing sleeve (8d). The distal rim section of the dose setting element encloses the proximal rim of the housing and is connected to the housing via notch (4e) to from an axially fixed but rotatable connection to the housing, e.g. the dose setting element (8) can rotate in both the dial up and the dial down direction without any axial movement. The inner housing sleeve (8d) has at its distal end a fourth coupling structure (8e) which rotationally couples the dose setting element (8) with a drive sleeve (11) having a fifth coupling structure (11a). The fourth and fifth coupling structures (8e, 11a) are preferably shaped as longitudinal protrusions and keyways that can slide with respect to each other and form the a first coupling (8e,11a). In the coupled state, rotations of the dose setting element are transmitted to the drive sleeve (11) and in the decoupled state, the drive sleeve (11) can rotate with respect to the dose setting element (8). The proximal rim section (8a) encloses a release button (12) which is shaped as a sleeve that is rotationally coupled to the dose setting element but can axially slide between a non-actuated or proximal position and an actuated or distal position. The release button preferably has longitudinal projections on the outside that fit into complementary recesses or keyways on the inside of the proximal rim section (8a) of the dose setting element. The release button (12) is closed at the proximal end by a cap (13) so that a user can push the release button from the non-actuated into the actuated position. Rotations of the dose setting element are always transferred to the release button, independent on whether the release button is in the non-actuated or the actuated position. A release spring (14) is present between the dose setting element and the release button and the spring forces intend to bring the release button into the non-actuated position. The release button (12) has an eighth coupling structure (12a) preferably located at the distal end which can be coupled or decoupled from a ninth coupling structure (4j) present at the inside of the housing (4). The two coupling structures (12a) and (4j) form a third coupling (12a,4j) which couples the release button (12) and dose setting element (8) to the housing when the release button moves from the non-actuated to the actuated position.

A power package (16) is functionally positioned between the release button (12) and the drive sleeve (11), which provides the energy needed for driving the piston rod. Preferably, the power package (16) is a spring element, preferably a compression or torsion spring or a spiral spring. One end of the spring element is preferably attached to the release button (12) whereas the other end is connected to the proximal end pin (11b) of the drive sleeve (11). The spring element can be pre-stressed or is stressed during the setting of a dose. In either case, the energy of the spring element (16) is operatively coupled between the drive sleeve (11) and the release button (12). Since the release button (12) is rotationally coupled to the dose setting element, potential energy cannot be released when the first coupling (8e,11a) between the drive sleeve (11) and the dose setting element (8) is in the coupled position.

The drive sleeve (11) comprises a proximal sleeve part (11c) connected to the end pin (11b). A notch (11d) axially fixates the drive sleeve to the release button to form a bearing between the release button- dose setting element assembly and the drive sleeve. Thus axial movements of the release button are transferred into axial movements of the drive sleeve. The drive sleeve (11) has longitudinal protrusions (11e) on the outside that match corresponding grooves on the inside of the scale drum (7), such that the scale drum is rotationally secured but axially slidable with respect to the drive sleeve. Any rotation of the dose setting element (8) during dose setting is transferred to the drive sleeve (11) via first coupling (8e, 11a) and from the drive sleeve (11) to the scale drum (7). The scale drum (7) is threadedly engaged with the housing and the outer threading (7b) of the scale drum matches an internal threading or thread segment of the housing and, as a consequence, the scale drum (7) axially shifts during dose setting. The maximum dose and the minimum dose (zero dose) that can be set is defined by axial and/or radial stops present at the scale drum (7) and the housing (4). During the dose setting process, the dose setting element is rotated in a first rotation direction for increasing a dose and in a second rotation direction for decreasing a set dose. The user can view the set dose printed on the outside of the scale drum through the viewing window (6). Thus during dose setting the scale drum axially shifts from the zero dose to the set dose position. During dose delivery, the drive sleeve (11) and scale drum rotate in the second rotation direction and the scale drum (7) returns from the set-dose position to the zero dose position defined by the axial and or radial stops between the scale drum and the housing.
The sleeve part (11c) of the drive sleeve has longitudinal grooves (11f) on the inside that match longitudinal protrusions (1i) on the outside of the first part or outer sleeve (1). The drive sleeve (11) and the first part (1) are rotationally coupled and axially slidable with respect to each other engaged. Any rotation in the first and/or second rotation direction during dose setting is also transferred to the first part (1) and the rotation in the second rotation direction during dose delivery is transferred to the first part (1). The drive sleeve (11) has a sixth coupling structure (11g) on the inside, shaped as longitudinal protrusions/keys that match a seventh coupling structure (2o) preferably designed as longitudinal grooves/keyways on the distal end of the second part (2). The sixth and seventh coupling structures form second coupling (2o, 11g) and when the second coupling (2o, 11g) is closed, initiated by an axial shift of the drive sleeve (11) in the distal direction, for example by pressing the release button (12), then the drive sleeve (11) and the first part (1) are rotationally locked with respect to each other and any rotation of the drive sleeve (11) is transferred to the second part (2).
The second part or coupling sleeve (2) is connected to the housing via a bearing (4i, 2p) comprising a circumferential rim (4i) on the inside of the inner housing sleeve (4g) and a circumferential notch (2p) located at the distal end of the second part (2). The bearing (4i,2p) ensures that the second part can rotate without axial movements with respect to the housing (4). A reverse lock sleeve (17) is coaxially arranged around the second part (2) and the reverse lock sleeve is splined to the outside surface of the second part such that the reverse lock sleeve (17) and the second part (2) are in a rotationally secured engagement while the reverse lock sleeve can axially move. The engagement between the two parts is preferably designed as longitudinal notches and protrusions. At the distal end of the reverse lock sleeve (17) is a one way ratchet system shaped as an asymmetric toothing (17a) present at the distal rim of the reverse lock sleeve in combination with a complementary toothing (2q) present at the proximal rim of the inner housing sleeve (4g). The one way ratchet system is biased by a reverse lock spring (18) which biases the one way ratchet system such that one rotation direction, preferably the first rotation direction for increasing a dose is prevented, whereas the opposite rotation direction or second rotation direction is allowed and the reverse lock sleeve ratchets over the complementary teething to produce audible clicks. The coupling sleeve thus can rotate in the second rotation direction only for delivery of a set dose after a minimum torque has been overcome of the one-way ratchet system. In the first rotation direction, the one-way ratchet prevents rotation of the reverse lock sleeve and the second part that is splined to the reverse lock sleeve.
During dose delivery, the second part rotates in the second rotation direction and drives a piston rod (9) in the distal direction for dose delivery. In the embodiment shown, the piston rod (9) is splined to the
Second part (2) in a non-rotatable but slidable engagement, for example though longitudinal grooves (9b) in the piston rod that match keys on the inside of the coupling sleeve. The piston rod has furthermore a threading (9a) on the outside that matches an internal threading (4h) of the housing and rotation of the second part (2) results in rotation and advancement of the piston rod through the housing. The second part (2) or coupling sleeve has a distal surface (21) shaped as a flange and protruding outwardly. On the proximal surface of the flange, the fourth coupling member (2m) is present as a toothing pointing in the proximal direction. The proximal flange or proximal surface (2i) is located, in the shown example, on a separate sleeve (2r) that can be assembled and attached to the main body of the second part (2), for example using a snap-fit connection that axially and rotationally secures the separate sleeve (2r) to the main body. The second coupling structure (2j) is present at the distal end of the sleeve as a toothing pointing in the distal direction. The modular approach is selected here to facilitate the mounting of the third part (3) between the two flanges of the second part (2), however functionally, the main body of the second part (2) and the sleeve (2r) behave as a single part. The two flanges are axially spaced apart and the third part (3) or stop sleeve is located between the two flanges and can shift between the proximal and distal positions during dose setting in the zig-zag type of motion described previously.

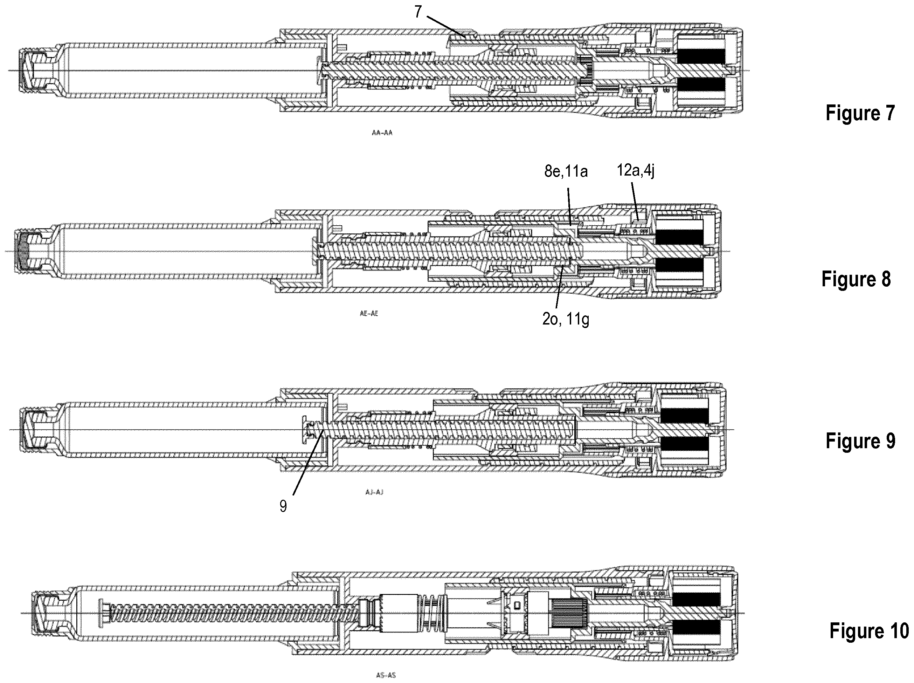

The functioning of the assembled device is described below. In the initial state, the first coupling (8e, 11a) is closed, the second coupling (2o, 11g) is open and the third coupling (12a, 4j) is open. A user grabs the device with the dose setting element (8) and rotates the dose setting element in the first rotation direction with respect to the housing for setting a dose that can be viewed through the viewing window (6). The rotation is transferred to the drive sleeve (11) via the first coupling (8e, 11a) which is closed. The rotation of the drive sleeve (11) is transferred to the scale drum (7) which axially slides with respect to the housing and the set dose can be viewed through the window in the housing (Figure 7). The drive sleeve (11) slaves the first part via the sliding connection between the grooves (11f) and the protrusions (1i). The first part rotates versus the non rotating second part (2). The second coupling (2o, 11g) is open and the second part is engaged with the housing (4) via the reverse lock sleeve (17) which prevents rotation of the second part in the first rotation direction and a certain torque for dose delivery needs to be overcome for rotation in the second rotation direction. The ratchet system is designed such that the torque needed to activate the ratchet is above any parasitic torques in the second rotation direction, for example due to the coupling-decoupling sequences that the third part exerts on the second part. Thus during dose setting the second part does not rotate with respect to the rotating first part. The stop sleeve or third part (3) is rotated by the first part (1) with a given transmission ratio due to the interactions between the guiding elements (1c, 1d), the steering means (3a) and the axial and rotational shifts of the stop sleeve, as described above in detail and shown in Figures 1-5. The third part is rotated over a number of discrete angular steps corresponding to the dose currently set. A dose setting click mechanism is functionally positioned between the rotating parts, for example the dose setting member(8) or the drive sleeve (11) and the non-rotating parts, for example the housing (4) or the second part (2).

After a dose has been and/or corrected, the set dose is dispensed from the device. The user pushes the release button (12) from the non-actuated into the actuated position (Figure 8). A number of coupling sequences can be envisaged, for example first the third coupling (12a, 4j) is coupled to rotationally lock the dose setting element- release button assembly to the housing. Then the second coupling (2o,11g) is moved from the decoupled to the coupled position to bring the drive sleeve (11) in a rotationally locked engagement with the second part or coupling sleeve (2). Finally the first coupling (8e,11a) is decoupled and the spring (16) is functionally positioned between the housing and drive sleeve (11) which starts rotating. Another coupling sequence could be to first couple the second part (2) to the drive sleeve (11) by closing the second coupling (2o, 11g), then couple the dose setting member (8) to the housing by closing the third coupling (12a, 4j) and finally release the potential energy of the spring (16) by opening the first coupling (8e, 11a). During actuation of the release button, the release button (12) dose setting element (8) are rotationally coupled to the housing and the second part (2) is coupled to the drive sleeve and finally the energy of the power package is released. The drive sleeve starts rotating in the second rotation direction and slaves the scale drum (7), the first part (1) and the second part (2). The scale drum rotates and axially shifts back to the zero dose position. The rotation of the second part (2) is transferred into axial movement of the piston rod to expel the dose set (Figure 9). The first part (1), the second part (2) and the third part (3) all co-rotate without any relative rotations and thus the angular position of the third part representing the dosage set is preserved. The reverse lock sleeve rotates together with the second part (2) and the one-way ratchet system produces audible dose delivery clicks. After the dose has been delivered, the user releases the release button and the resilient forces of the release spring move the release button in the proximal direction, decouple the third coupling (12a, 4j, couple the first coupling (2o, 11g) and decouple the second coupling (2o, 11g).

After repeated dose setting and dose dispensings, the remaining amount of medication is below the maximum value that can be set with the device. The stop sleeve (3) has been rotated over an angle that is still below the specific angle. During the setting of the last dose, the back and forth movement of the stop sleeve is halted when the first and/or second coupling members are in the stopping position and cannot axially slide anymore. As a consequence, rotational torque in the dial up direction is transferred from the dose setting element to the drive sleeve and from the drive sleeve to the first element. The rotation of the latter is blocked by the stop sleeve and consequently torque is transferred from the stop sleeve (3) to the second part (2) and from the second part to the housing via the one-way ratchet system. Once the stop sleeve (3) is in the stopping position, the opposite rotation in the second rotation direction for dose correction or dose delivery is allowed. A cross section of the injection device with the third part in the stopping position is shown in Figure 10.

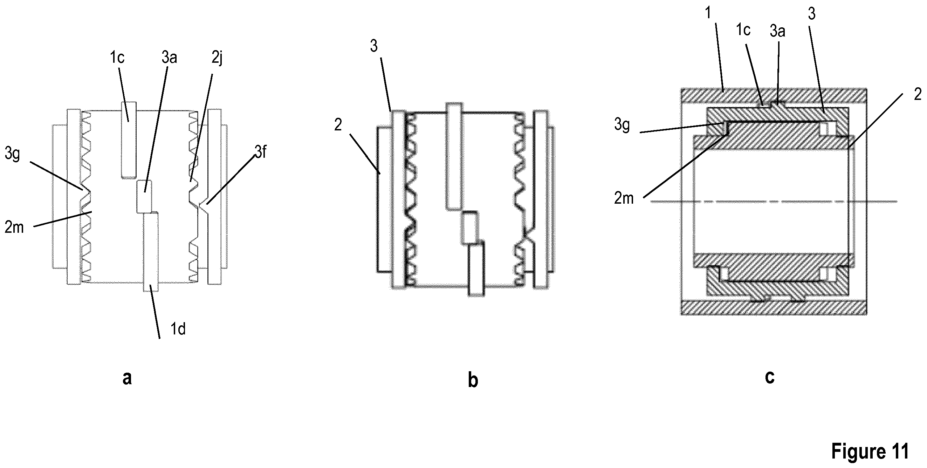

The dose limiting device according to a second embodiment of the invention is shown in Figure 11. The first part (1) or outer sleeve having the two guiding ribs on the inside is essentially identical to the first embodiment. The arcs or guiding elements axially rotate the third part or stop sleeve due to the interaction of the steering means (3a) on the outside of the stop sleeve with the arc shaped guiding ribs (1c, 1d, Figure 11a). The third part or stop sleeve in the second embodiment surrounds the second part or coupling sleeve (2), Figure 11c. Thus the stop sleeve has on the outside the guiding means and on the inside the first and third coupling members (3f, 3g). The axial shift of the third part is restricted by the coupling of the first or second couplings between the first and second coupling members or the third and fourth coupling members. The stop member is arranged on the second part and prevents further rotation and/or axial shift of the third part thereby limiting the dose setting (Figure 11b). The second part or coupling sleeve has a rotational axis going through an opening in the third part or stop sleeve (3).

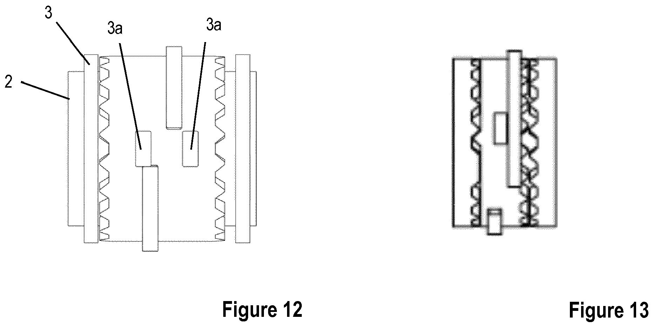

The dose limiting mechanism according to a third embodiment of the invention is shown in Figure 12. The functioning is essentially identical to the first or second embodiments of the invention, however the third part (3) has two steering means projecting outwards which implies that in the stopping position the loads are transferred from the first part to the third part via the two steering means. As a consequence, the contact pressure between the end surfaces of the guiding ribs of the first part and the steering means of the third part are lowered which reduces the strength requirements on the materials, for example plastic materials used. This might open options to use less strong and/or less expensive materials or on the other hand to transfer higher torques without any plastic deformation of the contact surfaces in the stopping position.

The dose limiting mechanism according to a fourth embodiment of the invention is shown in Figure 13. The functioning is essentially identical to the first embodiment but the width of the gap between the proximal and distal flanges of the second part has been narrowed such that the third part is either a ring (as shown) or can even be reduced to a guiding pin on the inside of the stop sleeve (not shown).

### List of reference signs

- 1: First part or outer sleeve
- 1a: Proximal rim outer sleeve
- 1b: Distal rim outer sleeve
- 1c: Guiding element, arc shaped guiding rib
- 1d: Guiding element, arc shaped guiding rib
- 1e: End surface guiding rib
- 1f: End surface guiding rib
- 1g: Distal circumferential surface
- 1h: Proximal circumferential surface
- 1i: Longitudinal protrusion

- 2: Second part or coupling sleeve
- 2a: Proximal part coupling sleeve
- 2b: Distal part coupling sleeve
- 2c: Connecting arm proximal part
- 2d: Connector proximal part
- 2e: Connecting arm distal part
- 2f: Connector distal part
- 2g: Opening
- 2h: Proximal sleeve of coupling sleeve
- 2i: Distal surface
- 2j: Second coupling member, circular toothing, sloped structures
- 2k: Distal sleeve of coupling sleeve
- 2l: Proximal surface
- 2m: Fourth coupling member, circular toothing, sloped structures
- 2n: Stop member
- 2o: Seventh coupling structure
- 2p: Notch
- 2q: Toothing
- 2r: Proximal flange

- 3: Third part or stop sleeve
- 3a: Steering means, cam
- 3b: second or short surface cam
- 3c: second or short surface cam
- 3d: first or long surface cam; distal
- 3e: first or long surface cam; proximal
- 3f: first coupling member, tooth, sloped structure
- 3g: third coupling member, tooth, sloped structure

- 4: Housing
- 4a: Longitudinal axis housing
- 4b: Outer housing sleeve
- 4c: Snap fit connector
- 4d: Circumferential rim
- 4e: Notch
- 4f: Circumferential rim
- 4g: Inner housing sleeve
- 4h: Internal thread segment
- 4i: rim
- 4j: Ninth coupling structure
- 4k: Internal thread segment

- 5: Cartridge holder
- 5a: Needle connector
- 5b: Protrusion

- 6: Viewing window

- 7: Scale drum
- 7a: Dose scale
- 7b: Outer thread

- 8: Dose setting element
- 8a: Proximal rim section
- 8b: Distal rim section
- 8c: Rim
- 8d: Inner housing sleeve
- 8e: Fourth coupling structure

- 9: Piston rod
- 9a: External threading piston rod
- 9b: Longitudinal keyways piston rod

- 10: Cartridge

- 11: Drive sleeve
- 11a: Fifth coupling structure
- 11b: End pin
- 11c: Sleeve part
- 11d: Notch
- 11e: Longitudinal protrusion outside
- 11f: Longitudinal grooves inside
- 11g: Sixth coupling structure

- 12: Release button
- 12a: Eight coupling structure

- 13: Cap

- 14: Release spring

- 16: Power package, spring

- 17: Reverse lock sleeve
- 17a: Asymmetric toothing

- 18: Reverse lock spring

- (8e,11a): First coupling
- (20, 11g): Second coupling
- (12a,4i): Third coupling
- (4i, 2p): Bearing

## Claims

1. A dose limiting mechanism for an injection device for injecting at least one dose of medication from a reservoir (10) comprising:
a housing (4) with a distal and a proximal end, the housing having a longitudinal axis (4a) whereby the reservoir (10) is located at the distal end of the housing,
a first part (1) which is rotated over a first angle during setting of a dose,
a second part (2) which does not rotate during setting of a dose,
a third part (3) which is operatively coupled to the first part (1) such that during each revolution of the first part, the third part (3) is rotated during the setting of a dose by the first part (1) over at least one discrete angular step which is a fraction of the first angle,
the rotation axis of the first, second and third part are approximately around and/or parallel to the longitudinal axis (4a) of the housing (4),
the second part (2) comprises a stop member (2n) which prevents further rotation of the third part (3) when the third part (3) has been rotated over a second angle, thereby limiting the dose setting to a predetermined amount of medication present in the reservoir (10),
**characterized by** that the third part (3) is urged to axially shift back and forth during dose setting between a proximal and a distal position due to engagement of coupling members (3f, 2j, 3g, 2m) present at the third part (3) and the second part (2) the axial shifts are urged by sloped structures which are present at or part of the coupling members (3f, 2j, 3g, 2m) and the interaction of two engaging coupling members which slide across each other urge the axial movement of the third part during rotation over the discrete angular step whereby during each axial shift the third part is rotated over one discrete angular step,
wherein the first part (1) is an outer sleeve axially fixed with respect to the housing (4) having at least two guiding elements (1c, 1d) and the at least two guiding elements (1c, 1d) guide the third part (3) during each revolution such that the third part (3) is rotated over the discrete angular step and is axially secured in the proximal or distal position,
wherein the third part (3) has a steering means (3a) wherein the guiding elements (1c, 1d) of the first part (1) abut the steering means such that rotation of the first part (1) is transmitted to the third part (3).

2. The dose limiting mechanism of claim 1, whereby during rotation over each discrete angular step the third part (3) in its proximal or distal position is coupled to, or decoupled from the second part (2) and is coupled to or decoupled from the second part (2) to the other one of the proximal or distal position.

3. The dose limiting mechanism of claim 1, whereby the second part (2) is operatively coupled to the housing (4) such that rotation of the second part (2) in one rotation direction corresponding to dose increase is prevented and rotation in the opposite direction is allowed.

4. The dose limiting mechanism of claim 3, whereby a reverse lock sleeve (17) is functionally positioned between the second part (2) and the housing (4) whereby the reverse lock sleeve (17) is rotationally coupled to the second part (2) and coupled to the housing (4) via a one-way ratchet system.

5. The dose limiting mechanism of claim 1, whereby the at least two guiding elements (1c, 1d) are arc shaped guiding ribs circumferentially arranged on the inside of the outer sleeve and the at least two arc shaped ribs are positioned axially displaced along the longitudinal axis with respect to each other and the at least two arc shaped ribs are angularly positioned with respect to each other to leave at least two arc shaped gaps between the at least two arc shaped ribs.

6. The dose limiting mechanism of claim 5, whereby the third part (3) preferably is a stop sleeve, and which stop sleeve has a steering means (3a) which can be operatively coupled to one of the at least two arc shaped ribs of the outer sleeve such that the stop sleeve is rotated during each revolution of the first part (1) and axially secured in the proximal or distal position.

7. The dose limiting mechanism of claim 6, whereby the steering means (3a) is an arc shaped cam fixed on the outside of the stop sleeve with an arc length at the most equal to the arc shaped gaps of the outer sleeve such that the stop sleeve can be rotationally coupled to the first part (1) by one of the at least two arc shaped ribs and whereby the arc shaped gaps allow for axial shifts of the arc shaped cam.

8. The dose limiting mechanism of claim 1, whereby the third part (3) is coaxially arranged around or surrounds the second part (2) and/or is located between a proximal (2i) and a distal (21) flange of the second part (2) such that the axial movements of the third part (3) between the proximal position and the distal position are restricted by the flanges, or whereby the third part (3) is coaxially arranged around the second part (2) and whereby the second part (2) has a track and the third part a track follower such that the axial movements of the third part between the proximal position and the distal position are restricted by the track and the track follower.

9. The dose limiting mechanism of claim 8, whereby the third part (3) has at least one first coupling member (3f) which can be coupled or decoupled to a second coupling member (2j) located at the second part (2) whereby the third part (3) has at least one third coupling member (3g) which can be coupled or decoupled to a fourth coupling member (2m) located at the second part (2) such that relative rotations between the third part (3) and the second part (2) are restricted when the third part (3) is in the proximal or distal position and the first and second coupling structures (3f, 2j) are coupled or the third and fourth coupling structures (3g, 2m) are coupled.

10. The dose limiting mechanism of claim 9, whereby the at least one first coupling member (3f) of the third part (3) comprises sloped structures, preferably at least one tooth with two slopes and which is located at the proximal rim and pointing in the proximal direction, and which fits into the second coupling member (2j) which comprises sloped structures, preferably designed as a teething located at the proximal flange (2i) of the second part (2) and which points in the distal direction, and whereby the third coupling member (3g) of the third part (3) comprises sloped structures, preferably at least one tooth with two slopes located at the distal rim pointing in the distal direction which fits into the fourth coupling member (2m) comprising sloped structures, preferably designed as a teething located at the distal flange (21) of the second part and which points into the proximal direction.

11. The dose limiting mechanism of claim 10, whereby the first and second coupling members (3f, 2j) are decoupled from the coupled position when the at least two guiding elements (1c, 1d) rotate the third part (3) over the discrete angular step, whereby the third and fourth coupling members (3g, 2m) are coupled from the decoupled position, whereby the third part (3) simultaneously shifts from the proximal to the distal position due to the interaction of the sloped structures.

12. The dose limiting mechanism of claim 8, whereby the proximal or distal flange (2i, 21) comprises the stop member (2n) which prevents axial shifts of the third part (3) when the first or third coupling structure (3f, 3g) abuts the stop element (2n) and the third part (3) has been rotated over the second angle, thereby limiting the dose setting to a predetermined amount of medication present in the reservoir (10).

13. An injection device for injecting at least one dose of medication having the dose limiting mechanism of one of the previous claims whereby a dose setting element (8) is operatively coupled to the first element (1) such that rotation of the dose setting element (8) is transferred to the first part (1) and whereby the second part (2) is operatively engaged with a piston rod (9) such that a rotation of the second part (2) advances the piston rod (9) for injecting a dose of medication.

14. An injection device according to claim 13, wherein the second part (2) is splined to the piston rod (9) such that the piston rod (9) and the second part (2) are in a non-rotatable but slidable engagement and wherein the piston rod (9) has an external thread (9a) that is in engagement with an internal threading (4h) of the housing (4) such that a rotation of the second part (2) rotates and advances the piston rod (9) for delivery of a dose of medication from the reservoir (10).

15. An injection device according to claim 13, wherein the piston rod (9) has an external thread (9a) that is threadedly engaged with the second part (2) and wherein the piston rod (9) is non-rotatable and axially slidable engaged with respect to the housing (4) such that a rotation of the second part (2) advances the piston rod (9) for delivery of a dose of medication from the reservoir (10)

## Patentansprüche

1. Dosis beschränkender Mechanismus für ein Injektionsgerät zum Injizieren mindestens einer Dosis einer Medikation aus einem Reservoir (10), welcher umfasst:
ein Gehäuse (4) mit einem distalen und einem proximalen Ende, worin das Gehäuse eine Längsachse (4a) aufweist, worin das Reservoir (10) an dem distalen Ende des Gehäuses angeordnet ist,
ein erstes Teil (1), das während des Einstellens einer Dosis über einen ersten Winkel gedreht wird,
ein zweites Teil (2), das sich während des Einstellens einer Dosis nicht dreht,
ein drittes Teil (3), das mit dem ersten Teil (1) betreibbar gekoppelt ist, so dass während jeder Umdrehung des ersten Teils während des Einstellens einer Dosis durch das erste Teil (1), das dritte Teil (3) über mindestens einen separaten Winkelschritt, der ein Anteil des ersten Winkels ist, gedreht wird,
worin die Drehachsen des ersten, zweiten und dritten Teils näherungsweise um und/oder parallel der Längsachse (4a) des Gehäuses (4) sind,
worin das zweite Teil (2) ein Stoppelement (2n) umfasst, das eine weitere Drehung des dritten Teils (3) verhindert, wenn das dritte Teil (3) über einen zweiten Winkel gedreht wurde, wodurch die Dosiseinstellung auf einer bestimmten Menge der in dem Reservoir (10) vorliegenden Medikation beschränkt wird,
**dadurch gekennzeichnet, dass**
das dritte Teil (3) dazu gebracht wird, während des Einstellens einer Dosis zwischen einer proximalen und einer distalen Position durch Eingriffn von Kupplungselementen (3f, 2j, 3g, 2m), die an dem dritten Teil (3) und dem zweiten Teil (2) vorliegen, axial rückwärts und vorwärts zu versetzen, wobei die axialen Versetzungen durch abgeschrägte Aufbauten bewirkt werden, die an den Kupplungselementen (3f, 2j, 3g, 2m) vorgesehen sind oder Teil von diesen sind und wobei der Eingriff von zwei in Eingriff kommenden Kupplungselementen, die übereinander gleiten, die axiale Bewegung des dritten Teils während einer Drehung über den separaten Winkelschritt treibt, wobei während jeder axialen Versetzung das dritte Teil über einen separaten Winkelschritt gedreht wird,
worin das erste Teil (1) eine äußere Hülse ist, die mit Bezug zu dem Gehäuse (4) axial befestigt ist, mindestens zwei Führungselemente (1c, 1d) aufweist und worin die mindestens zwei Führungselemente (1c, 1d) während jeder Umdrehung den dritten Teil (3) führen, so dass das dritte Teil (3) über den separaten Winkelschritt gedreht wird und axial in der proximalen oder der distalen Position gesichert ist,
worin das dritte Teil (3) ein Steuerungsmittel (3a) aufweist, worin die Führungselemente (1c, 1d) des ersten Teils (1) gegen die Steuerungsmittel stoßen, so dass eine Drehung des ersten Teils (1) zu dem dritten Teil (3) übertragen wird.

2. Dosis beschränkender Mechanismus nach Anspruch 1, worin das dritte Teil (3) während einer Drehung über jeden separaten Winkelschritt in seiner proximalen oder distalen Position gekoppelt wird mit, oder entkoppelt wird von dem zweiten Teil (2) und gekoppelt wird mit oder entkoppelt wird von dem zweiten Teil (2) in die andere proximale bzw. distale Position.

3. Dosis beschränkender Mechanismus nach Anspruch 1, worin das zweite Teil (2) mit dem Gehäuse (4) betreibbar gekoppelt ist, so dass die Drehung des zweiten Teils (2) in eine der Dosissteigerung entsprechenden Drehrichtung verhindert wird und eine Drehung in die entgegengesetzte Richtung möglich ist.

4. Dosis beschränkender Mechanismus nach Anspruch 3, worin eine Rückwärts-Sperrhülse (17) zwischen dem zweiten Teil (2) und dem Gehäuse (4) funktional angeordnet ist, worin die Rückwärts-Sperrhülse (17) mit dem zweiten Teil (2) drehbar gekoppelt ist und mit dem Gehäuse (4) über ein Einweg-Ratschensystem gekoppelt ist.

5. Dosis beschränkender Mechanismus nach Anspruch 1, worin die mindestens zwei Führungselemente (1c, 1d) bogenförmige Führungsrippen sind, die auf der Innenseite der äußeren Hülse umlaufend angeordnet sind und worin die mindestens zwei bogenförmigen Rippen entlang der Längsachse im Bezug zueinander axial versetzt angeordnet sind und worin die mindestens zwei bogenförmigen Rippen im Bezug zueinander winkelig angeordnet sind, um mindestens zwei bogenförmige Aussparungen zwischen den mindestens zwei bogenförmigen Rippen zu belassen.

6. Dosis beschränkender Mechanismus nach Anspruch 5, worin das dritte Teil (3) vorzugsweise eine Stopphülse ist, und worin die Stopphülse ein Steuerungsmittel (3a) aufweist, das mit einem der mindestens zwei bogenförmigen Rippen der äußeren Hülse betreibbar gekoppelt werden kann, so dass die Stopphülse während jeder Umdrehung des ersten Teils (1) gedreht wird und axial in der proximalen oder distalen Position gesichert wird.

7. Dosis beschränkender Mechanismus nach Anspruch 6, worin das Steuerungsmittel (3a) ein bogenförmiger Nocken ist, der auf der Außenseite der Stopphülse mit einer Bogenlänge möglichst gleich den bogenförmigen Aussparungen der äußeren Hülse befestigt ist, so dass die Stopphülse mit dem ersten Teil (1) durch eine der mindestens zwei bogenförmigen Rippen drehbar gekoppelt werden kann und worin die bogenförmigen Aussparungen axiale Versetzungen des bogenförmigen Nocken ermöglichen.

8. Dosis beschränkender Mechanismus nach Anspruch 1, worin das dritte Teil (3) um den zweiten Teil (2) koaxial angeordnet ist oder diesen umgibt und/oder zwischen einem proximalen (2i) und einem distalen (2l) Flansch des zweiten Teils (2) angeordnet ist, so dass die axialen Bewegungen des dritten Teils (3) zwischen der proximalen Position und der distalen Position durch die Flansche beschränkt werden, oder worin das dritte Teil (3) koaxial um den zweiten Teil (2) angeordnet ist und worin das zweite Teil (2) eine Spur und das dritte Teil einen Spurfolger aufweist, so dass die axialen Bewegungen des dritten Teils zwischen der proximalen Position und der distalen Position durch die Spur und den Spurfolger beschränkt werden.

9. Dosis beschränkender Mechanismus nach Anspruch 8, worin das dritte Teil (3) mindestens ein erstes Kupplungselement (3f) aufweist, das mit einem zweiten an dem zweiten Teil (2) angeordneten Kupplungselement (2j) gekoppelt oder entkoppelt werden kann, worin das dritte Teil (3) mindestens ein drittes Kupplungselement (3g) aufweist, das mit einem an dem zweiten Teil (2) angeordneten vierten Kupplungselement (2m) gekoppelt oder entkoppelt werden kann, so dass relative Drehungen zwischen dem dritten Teil (3) und dem zweiten Teil (2) beschränkt werden, wenn das dritte Teil (3) in der proximalen oder distalen Position ist und der erste und zweite Kupplungsaufbau (3f, 2j) gekoppelt sind oder der dritte und vierte Kupplungsaufbau (3g, 2m) gekoppelt sind.

10. Dosis beschränkender Mechanismus nach Anspruch 9, worin das mindestens eine erste Kupplungselement (3f) des dritten Teils (3) schräge Aufbauten umfasst, vorzugsweise mindestens einen Zahn mit zwei Schrägen und das an dem proximalen Rand angeordnet ist und das in die proximale Richtung zeigt, und das in das zweite Kupplungselement (2j) passt, das schräge Aufbauten umfasst, vorzugsweise ausgestaltet als eine Bezahnung, die an dem proximalen Flansch (2i) des zweiten Teils (2) angeordnet ist und das in die distale Richtung zeigt, und worin das dritte Kupplungselement (3g) des dritten Teils (3) schräge Aufbauten umfasst, vorzugsweise mindestens einen Zahn mit zwei an dem distalen Rand angeordneten Schrägen, der in die distale Richtung zeigt, und der in das vierte Kupplungselement (2m) passt, das schräge Aufbauten umfasst, vorzugsweise ausgestaltet als eine an dem distalen Flansch (21) des zweiten Teils angeordnete Bezahnung und die in die proximale Richtung zeigt.

11. Dosis beschränkender Mechanismus nach Anspruch 10, worin das erste und zweite Kupplungselement (3f, 2j) von der Kupplungsposition entkoppelt sind, wenn die mindestens zwei Führungselemente (1c, 1d) des dritten Teils (3) über den separaten Winkelschritt drehen, worin das dritte und vierte Kupplungselement (3g, 2m) von der Entkopplungsposition gekoppelt werden, und wobei gleichzeitig das dritte Teil (3) durch Eingriff der schrägen Aufbauten von der proximalen in die distale Position versetzt wird.

12. Dosis beschränkender Mechanismus nach Anspruch 8, worin der proximale oder distale Flansch (2i, 21) das Stoppelement (2n) umfasst, das axiale Versetzungen des dritten Teils (3) verhindert, wenn der erste oder dritte Kupplungsaufbau (3f, 3g) gegen das Stoppelement (2n) stößt und das dritte Teil (3) über den zweiten Winkel gedreht wurde, wodurch das Einstellen der Dosis zu einer bestimmten Menge von in dem Reservoir (10) vorliegender Medikation beschränkt ist.

13. Injektionsgerät zum Injizieren mindestens einer Dosis einer Medikation, das einen Dosis beschränkenden Mechanismus eines der vorstehenden Ansprüche aufweist, wobei ein Dosis-Einstellelement (8) mit dem ersten Element (1) betreibbar gekoppelt ist, so dass eine Drehung des Dosis-Einstellelements (8) auf das ersten Teil (1) übertragen wird und worin das zweite Teil (2) mit einer Kolbenstange (9) betreibbar in Eingriff kommt, so dass eine Drehung des zweiten Teils (2) die Kolbenstange (9) zum Injizieren einer Dosis einer Medikation vortreibt.

14. Injektionsgerät nach Anspruch 13, worin der zweite Teil (2) mit der Kolbenstange (9) verkeilt ist, so dass die Kolbenstange (9) und das zweite Teil (2) in einer nicht-drehbaren jedoch gleitenden Eingriffnahme sind und worin die Kolbenstange (9) ein externes Gewinde (9a) aufweist, das mit einem internen Gewinde (4h) des Gehäuses (4) in Eingriff steht, so dass eine Drehung des zweiten Teils (2) die Kolbenstange (9) zur Abgabe einer Dosis einer Medikation aus dem Reservoir (10) dreht und vortreibt.

15. Injektionsgerät nach Anspruch 13, worin die Kolbenstange (9) ein externes Gewinde (9a) aufweist, das mit dem zweiten Teil (2) mit einem Gewinde versehen in Eingriff kommt und worin die Kolbenstange (9) nicht-drehbar ist und axial gleitend im Bezug zu dem Gehäuse (4) in Eingriff kommt, so dass eine Drehung des zweiten Teils (2) die Kolbenstange (9) zur Abgabe einer Dosis einer Medikation aus dem Reservoir (10) vortreibt.

## Revendications

1. Mécanisme de limitation de dose pour un dispositif d'injection pour injecter au moins une dose de médicament depuis un réservoir (10) comprenant :
un boîtier (4) avec une extrémité distale et une proximale, le boîtier ayant un axe longitudinal (4a) selon lequel le réservoir (10) se trouve au niveau de l'extrémité distale du boîtier,
une première partie (1) qui est en rotation sur un premier angle durant le réglage d'une dose,
une deuxième partie (2) qui n'est pas en rotation durant le réglage d'une dose,
une troisième partie (3) qui est fonctionnellement couplée à la première partie (1) de telle sorte que durant chaque révolution de la première partie, la troisième partie (3) est en rotation durant le réglage d'une dose par la première partie (1) sur au moins un palier angulaire discret qui est une fraction du premier angle,
les axes de rotation des première, deuxième et troisième parties sont approximativement autour de et/ou parallèles à l'axe longitudinal (4a) du boîtier (4),
la deuxième partie (2) comprend un élément d'arrêt (2n) qui empêche une rotation supplémentaire de la troisième partie (3) lorsque la troisième partie (3) a été entraînée en rotation sur un deuxième angle, limitant ainsi le réglage de dose à une quantité prédéterminée de médicament présent dans le réservoir (10),
**caractérisé en ce que** la troisième partie (3) est poussée pour se décaler axialement selon un mouvement de va-et-vient durant le réglage de dose entre une position proximale et une distale en raison de la mise en prise d'éléments de couplage (3f, 2j, 3g, 2m) présents au niveau de la troisième partie (3) et de la deuxième partie (2) les décalages axiaux sont poussés par des structures inclinées qui sont présentes au niveau ou font partie des éléments de couplage (3f, 2j, 3g, 2m) et l'interaction de deux éléments de couplage en prise qui coulissent l'un sur l'autre pousse le mouvement axial de la troisième partie durant la rotation sur le palier angulaire discret moyennant quoi durant chaque décalage axial la troisième partie est entraînée en rotation sur un palier angulaire discret,
dans lequel la première partie (1) est un manchon externe axialement fixé relativement au boîtier (4) ayant au moins deux éléments de guidage (1c, 1d) et les au moins deux éléments de guidage (1c, 1d) guident la troisième partie (3) durant chaque révolution de telle sorte que la troisième partie (3) est entraînée en rotation sur le palier angulaire discret et est axialement fixée dans la position proximale ou distale,
dans lequel la troisième partie (3) a un moyen d'orientation (3a) dans lequel les éléments de guidage (1c, 1d) de la première partie (1) butent contre le moyen d'orientation de telle sorte que la rotation de la première partie (1) est transmise à la troisième partie (3).

2. Mécanisme de limitation de dose selon la revendication 1, selon lequel durant la rotation sur chaque palier angulaire discret la troisième partie (3) dans sa position proximale ou distale est couplée à, ou découplée de la deuxième partie (2) et est couplée à ou découplée de la deuxième partie (2) à l'autre de la position proximale ou distale.

3. Mécanisme de limitation de dose selon la revendication 1, selon lequel la deuxième partie (2) est fonctionnellement couplée au boîtier (4) de telle sorte que la rotation de la deuxième partie (2) dans une direction de rotation correspondant à une augmentation de dose est empêchée et la rotation dans la direction opposée est permise.

4. Mécanisme de limitation de dose selon la revendication 3, selon lequel un manchon de blocage inverse (17) est fonctionnellement positionné entre la deuxième partie (2) et le boîtier (4) selon lequel le manchon de blocage inverse (17) et couplé en rotation à la deuxième partie (2) et couplé au boîtier (4) via un système de cliquet à sens unique.

5. Mécanisme de limitation de dose selon la revendication 1, selon lequel les au moins deux éléments de guidage (1c, 1d) sont des nervures de guidage en forme d'arc agencées circonférentiellement sur l'intérieur du manchon externe et les au moins deux nervures en forme d'arc sont positionnées axialement déplacées le long de l'axe longitudinal l'une par rapport à l'autre et les au moins deux nervures en forme d'arc sont positionnées angulairement l'une par rapport à l'autre pour laisser au moins deux espaces en forme d'arc entre les au moins deux nervures en forme d'arc.

6. Mécanisme de limitation de dose selon la revendication 5, selon lequel la troisième partie (3) est de préférence un manchon d'arrêt, et ledit manchon d'arrêt a un moyen d'orientation (3a) qui peut être fonctionnellement couplé à l'une des au moins deux nervures en forme d'arc du manchon externe de telle sorte que le manchon d'arrêt est entraîné en rotation durant chaque révolution de la première partie (1) et axialement fixé dans la position proximale ou distale.

7. Mécanisme de limitation de dose selon la revendication 6, selon lequel le moyen d'orientation (3a) est une came en forme d'arc fixée sur l'extérieur du manchon d'arrêt avec une longueur d'arc au plus égale aux espaces en forme d'arc du manchon externe de telle sorte que le manchon d'arrêt peut être couplé en rotation à la première partie (1) par l'une des au moins deux nervures en forme d'arc et selon lequel les espaces en forme d'arc permettent les décalages axiaux de la came en forme d'arc.

8. Mécanisme de limitation de dose selon la revendication 1, selon lequel la troisième partie (3) est coaxialement disposée autour de ou entoure la deuxième partie (2) et/ou se trouve entre une bride proximale (2i) et une distale (21) de la deuxième partie (2) de telle sorte que les mouvements axiaux de la troisième partie (3) entre la position proximale et la position distale sont restreints par les brides, ou selon lequel la troisième partie (3) est coaxialement agencée autour de la deuxième partie (2) et selon lequel la deuxième partie (2) a une piste et la troisième partie un suiveur de piste de telle sorte que les mouvements axiaux de la troisième partie entre la position proximale et la position distale sont restreints par la piste et le suiveur de piste.

9. Mécanisme de limitation de dose selon la revendication 8, selon lequel la troisième partie (3) a au moins un premier élément de couplage (3f) qui peut être couplé à ou découplé d'un deuxième élément de couplage (2j) situé au niveau de la deuxième partie (2) selon lequel la troisième partie (3) a au moins un troisième élément de couplage (3g) qui peut être couplé à ou découplé d'un quatrième élément de couplage (2m) situé au niveau de la deuxième partie (2) de telle sorte que les rotations relatives entre la troisième partie (3) et la deuxième partie (2) sont restreintes lorsque la troisième partie (3) est dans la position proximale ou distale et les première et deuxième structures de couplage (3f, 2j) sont couplées ou les troisième et quatrième structures de couplage (3g, 2m) sont couplées.

10. Mécanisme de limitation de dose selon la revendication 9, selon lequel l'au moins un premier élément de couplage (3f) de la troisième partie (3) comprend des structures inclinées, de préférence au moins une dent avec deux pentes et qui se trouve au niveau du bord proximal et pointant dans la direction proximale, et qui s'adapte dans le deuxième élément de couplage (2j) qui comprend des structures inclinées, de préférence conçu comme une denture située au niveau de la bride proximale (2i) de la deuxième partie (2) et qui pointe dans la direction distale, et selon lequel le troisième élément de couplage (3g) de la troisième partie (3) comprend des structures inclinées, de préférence au moins une dent avec deux pentes qui se trouve au niveau du bord distal pointant dans la direction distale qui s'adapte dans le quatrième élément de couplage (2m) comprenant des structures inclinées, de préférence conçu comme une denture située au niveau de la bride distale (21) de la deuxième partie et qui pointe dans la direction proximale.

11. Mécanisme de limitation de dose selon la revendication 10, selon lequel les premier et deuxième éléments de couplage (3f, 2j) sont découplés de la position couplée lorsque les au moins deux éléments de guidage (1c, 1d) entraînent en rotation la troisième partie (3) sur le palier angulaire discret, selon lequel les troisième et quatrième éléments de couplage (3g, 2m) sont couplés de la position découplée, selon lequel la troisième partie (3) se décale simultanément de la position proximale à distale en raison de l'interaction des structures inclinées.

12. Mécanisme de limitation de dose selon la revendication 8, selon lequel la bride proximale ou distale (2i, 21) comprend l'élément d'arrêt (2n) qui empêche les décalages axiaux de la troisième partie (3) lorsque la première ou troisième structure de couplage (3f, 3g) bute contre l'élément d'arrêt (2n) et la troisième partie (3) a été entraînée en rotation sur le deuxième angle, limitant ainsi le réglage de dose à une quantité prédéterminée de médicament présent dans le réservoir (10).

13. Dispositif d'injection pour injecter au moins une dose de médicament ayant le mécanisme de limitation de dose selon l'une des revendications précédentes selon lequel un élément de réglage de dose (8) est fonctionnellement couplé au premier élément (1) de telle sorte que la rotation de l'élément de réglage de dose (8) est transférée à la première partie (1) et selon lequel la deuxième partie (2) est fonctionnellement en prise avec une tige de piston (9) de telle sorte qu'une rotation de la deuxième partie (2) fait avancer la tige de piston (9) pour l'injection d'une dose de médicament.

14. Dispositif d'injection selon la revendication 13, dans lequel la deuxième partie (2) est solidarisée par cannelures à la tige de piston (9) de telle sorte que la tige de piston (9) et la deuxième partie (2) sont en prise non rotative mais coulissante et dans lequel la tige de piston (9) a un filetage externe (9a) qui est en prise avec un filetage interne (4h) du boîtier (4) de telle sorte qu'une rotation de la deuxième partie (2) entraîne en rotation et fait avancer la tige de piston (9) pour l'administration d'une dose de médicament depuis le réservoir (10).

15. Dispositif d'injection selon la revendication 13, dans lequel la tige de piston (9) a un filetage externe (9a) qui est en prise par filetage avec la deuxième partie (2) et dans lequel la tige de piston (9) est en prise non rotative et axialement coulissante par rapport au boîtier (4) de telle sorte qu'une rotation de la deuxième partie (2) fait avancer la tige de piston (9) pour l'administration d'une dose de médicament depuis le réservoir (10).
